# EUROPEAN PATENT SPECIFICATION

(11) **EP 2 632 547 B1**
(45) Date of publication and mention of the grant of the patent: **25.12.2019**
(21) Application number: 11805584.7
(22) Date of filing: 25.10.2011
(51) Int. Cl.: A61N 2/00, A61N 2/02, A61C 8/00

(54) **SYSTEM FOR ELECTRO-MAGNETIC CELLULAR TREATMENT**
SYSTEM ZU ELEKTROMAGNETISCHEN BEHANDLUNG VON ZELLEN
SYSTÈME DE TRAITEMENT CELLULAIRE ÉLECTROMAGNÉTIQUE

(30) Priority: 25.10.2010 RO 201001010; 03.02.2011 WO PCT/RO2011/000004; 09.05.2011 RO 201100444; 18.07.2011 WO PCT/RO2011/000019
(43) Date of publication of application: 04.09.2013
(73) Proprietor: Vladila, Bogdan Constantin, 050723 Bucharest (RO)
(72) Inventor: Vladila, Bogdan Constantin, 050723 Bucharest (RO)
(74) Representative: Tuluca, F. Doina
(86) International application number: PCT/IB2011/002804
(87) International publication number: WO 2012/093277

(56) References cited:
- EP-A1- 1 943 977
- EP-A2- 2 074 958
- WO-A1-00/09031
- WO-A1-02/056838
- WO-A1-02/102457
- WO-A1-2007/051339
- FR-A1- 2 510 400
- US-A- 3 915 151
- US-A- 4 671 768
- US-A1- 2002 039 718
- US-A1- 2007 005 042
- US-A1- 2007 111 163
- US-B1- 6 602 516

## Description

### Field of the Invention

The present invention relates to a system for improving tissue regeneration at the cellular level. More particularly, the present invention relates to a system using electro magnetic fields for improving tissue regeneration at the cellular level.

### Background of the Invention

In many varied fields of orthopedic and cosmetic procedures, lowering the period of healing of fractured bones and damaged tissues, as well as of the biointegration of implants, is a primary consideration.

The resorption of tissue and bone structure, affected by an implant or otherwise, is a continuous process which takes time. The reason is a lowering of the local blood microcirculation and, in the particular field of dentistry for instance, results in dental mobility. The level of tissue integration depends firstly on the development rate of new cells in the region of surgical trauma. If the flow of these cells is high, a rapid neovascularization of the region is produced and the cells can survive on the implanted material. The protoglycans layer may be reduced and the proportion of direct contact between bone and implant will increase in percentage. Normally, dead cells are immediately replaced by new progenitor cells, to be converted into osteoblast, cementoblast cells, etc, according to the specific character of that region.

Various technico - medical solutions have been developed for improving the biointegration of implants. The use of porous titanium, of zirconium oxide, the alloying of titanium with zirconium to obtain roxolid, are only a few known solutions for improving biocompatibility.

Continuous research and development both in connected and in far removed fields of application have opened surprising avenues of further improvement in cellular regeneration.

In 1952, Doctor Winfried Otto Schumann of the Faculty of Science in Munich succeeded in demonstrating the theory that the terrestrial space comprised between the earth surface and ionosphere behaves as a wave guide and at the same time as a resonance box. The frequency spectrum of this space ranges approximately between 6 and 50 Hz, with the main average value of 7.83 Hz. As life on earth was created within this space, all that we call a living organism was adapted to this frequency. Vital functions of organisms deteriorate when not subjected to this frequency over extended periods of time. This explains why astronauts' bodies suffer important disturbances during extra - terrestrial flight, such as parodontopathy and osteoporosis. These conditions have since been at least partly prevented by artificially generating the 7.83 Hz frequency on board spaceships. Scientists have since discovered that, besides the basic frequency vital for each organism to function, the internal organs and cells constituting them also react favourably to other frequencies, some of them vital for their restoration when they have been subjected to lesions or when suffering from certain affections.

Further, James Oschman demonstrated that each normal or pathological event carried on within any organism, produces modifications of the electromagnetic field generated by said organism. Based on these principles, devices have been built for monitoring the heart, brain activity or to precisely determine an ovulation period. It was also Oschman who demonstrated that the muscle activity generates electromagnetic impulses that stimulate the cells regeneration, starting from the attraction of undifferentiated mesenchymal cells, and that any affection, such as a lesion caused by surgery, determines the modification of the magnetic field in the region of tissue trauma. In other words, the modification in the number of capillaries is accompanied by an increase of the magnetic "reluctance" of adjacent tissues. This increase is also determined both by the minerals included in the tophus extant in that region, and by any local metal implants that disturb or prevent the normal, regenerating magnetic field resulting from the Schumman magnetic frequencies and the biological cell oscillations. From the aspects presented above, a conclusion may be drawn that the bio - integrability of an implant, impeded by the increase of the magnetic reluctance in the region due to wounding the tissues, due to tophus, microbes extant in the region as well as the insertion of any metal implants, can nevertheless be hastened by bringing the regenerating magnetic field to normal values.

Sisken and Walker have demonstrated that the frequencies of 2 Hz, 25Hz and 50 Hz stimulate nervous regeneration, which is useful in implantology if hypoesthesia occurs following a surgical procedure. Sisken and Walker have also demonstrated that a 7 Hz frequency stimulates osseous regeneration and that a 10 Hz frequency stimulates ligament regeneration, which is for instance useful in parodontology for lowering teeth mobility. Sisken and Walker have also demonstrated that the 15 Hz, 20 Hz and 72 Hz frequencies stimulate the reformation of capillaries, this action being necessary after any surgical intervention involving osseous or soft tissue graft. Herbert Frachlich demonstrated that an assembly of cells forming a tissue or organ has a specific frequency that regulates the physiology of said organ, and that if a large number of cells are affected, then the frequency cannot be emitted any longer and the disease or dysfunction occurs.

The prior art described above shows that a healthy organ contributes to maintaining the health in its neighbouring region, however a diseased organ cannot do this any longer and suitable steps should be taken to remedy the situation. Unfortunately, in the buccal cavity in particular, the natural regenerating field is reduced, since the microbes and minerals extant in the tophus composition "steal" from the regenerating field intensity in order to mineralise themselves. It is known that the phenomena of healing by magnetic oscillations occur at low amplitudes of the field, with a magnetic induction of about 10⁻⁹ to 10⁻¹⁰ Tesla.

International patent application WO 2009/04215 A1 discloses an electronic apparatus meant to hasten tissue healing in the knee region, by means of a low - frequency electromagnetic field. The apparatus belongs to a system meant to heal tissues and bones having different affections, and it is further provided with an electric circuit for magnetic field pulse control.

In order to solve the partial edentition problems there are known and applied a great variety of shapes of metal implants usually made of titanium, more or less alloyed, since this is sufficiently well tolerated by the human body, it is easily workable, has a good mechanical strength to compression, torsion and bending and, consequently, allows to achieve compound dental implants also called "in two surgical stages", which have the advantage of giving the doctor the opportunity of selecting the prosthetic work type favourable to the subject, allow to comply with the hibernation period necessary for integrating the implant and favours the mounting of parallel prosthetic blunts for carrying on thereon dental crowns perfectly adapted to said case.

Subsequently, more researchers ascertained that the osteointegration time of an implant diminishes in case of carrying out the same from a ceramic material, more exactly zirconium oxide, since, in comparison with titanium for example, this is better tolerated by the organism and at the same time has the quality of not favouring the formation of electric cells even if combined with different other metals. Practically, there exist only few subjects allergic to this material, this material being both biocompatible and bioinert. Then, the zirconium is preferred also with regard to the thermal conductivity. It does not conduct heat so that the thermal variations are not transmitted to the alveolar bone. This is extremely important particularly when the implant shall be adjusted by grinding right in the subject's mouth. Moreover, even if it does not conduct heat, the zirconium oxide conducts the regenerating low frequency oscillations existing in the buccal cavity very well.

We shall also add here the fact that both titanium and zirconium, with their alloys, are used in implantology also for the reason that both have the characteristic of not inhibiting the growth of osteoblasts, which are essential cells for osteointegration.

Given the high qualities of zirconium oxide in respect of the osteointegration, it was tried to achieve compound implants, consequently implants to be inserted in two surgical stages and made of this material. Unfortunately, this was not yet possible due to low zirconium oxide workability, characteristic that has as effect that certain fineness operations, among which threading, cannot practically be carried out for the time being in an optimum way, such as in metals. Consequently, materials with increased biocompatibility, such as zirconium oxide and other ceramics, do not allow to obtain a self-threading region at the implant tip, nor the carrying out of an internal thread resisting in time, through which the connection between the prosthetic blunt and the implant proper can be made. As such, the implants made of zirconium oxide are usually carried out as a single item - reason for which they are also called one-piece implants - and the construction of the dental crown thereon is very difficult. This is due to the fact that on such implants only cemented works can be done, at the moment there being practically impossible to carry *out* threaded works.

It has not been possible so far to carry out viable implants which should combine workability and the mechanical qualities of the titanium with zirconium biocompatibility.

It is to be mentioned that the JP 2009254547 (A) patent application of 2009.11.05 points out the fact that a composition comprising octacalcium phosphate can successfully contribute to osseous regeneration. Generalising, the researches carried out by numerous specialists demonstrated that there exist other mineral substances that help in normalising the cellular membrane level exchanges and enzymatic equilibrium regulating.

The patent application FR 2926460 of 22.01.2008, having Messers Gilles Gutierrez and Bogdan Vl 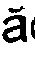 dil 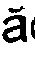 as authors, presents a first use of opal in dental surgery, with a view to accelerating the growth of osseous mass, disposed around an implant or around a tooth root. To this end, there is prepared a composition comprising natural or synthetic opal - in a ratio of 0.5 up to 20%, preferably between 1 and 10%- incorporated in a mass of easily polymerizable or fusible polymeric material. By the researches performed by the two authors, it has been ascertained that the existence of opal in this composition causes three times higher cell migration, particularly of osteoblasts, cementoblasts, fibroblasts or keratinocites, than in the default thereof. In other words, things happen as if opal would transmit a signal that calls said cells towards it. The solution can also be applied in the field of dental implants in order to succeed to improve the implant integration into the alveolar bone.

The same authors subsequently filed, on July 10, 2008, also in France, with no. 0803921/FR 2933610 another patent application entitled "Opal based composition for the treatment of dental affections". The composition includes an opal powder that is incorporated into the thermofusible polymer mass selected from polyolefins, such as polyethylenes or polypropylenes, alone or in admixture. From this mixture there is obtained a mould, customarized to each subject through a dental guard, with a view to periodontal regenerating. The obtained results mentioned by the authors within the application are very promising. It has to be added here that the application of the solution continues at present and the results are remarkable. The author of the present invention has treated more than 100 subjects so far by the use of such a guard.

The authors also demonstrated that the cell migration is carried out even if there does not exist a direct contact between opal - or a composition comprising opal - and the affected tissue. However, this migration is influenced by more factors, among which the subject's oral hygiene, age, material interposed between opal and the affected tissue and the opal concentration of a certain mixture. Then it was demonstrated that neither titanium nor zirconium hinder too much the signal transmitted by opal to the distance, signal that determines the cell migration. However, zirconium favors the transmission of this signal to a greater extent than the titanium. Even if, for a certain type of implant, the area occupied by the zirconium oxide cannot be too large, this is to be created, since zirconium is the one to favor the regenerating signal transmission better than any other metal. These experiments also reconfirmed the theory that the natural tooth itself seems to emit the signal which attacks towards it the progenitor cells with the purpose of regenerating what is destroyed due to the microbial activity. This regenerating signal intensity lowers with the age, and the tophus shields the transmission thereof. Unfortunately, the actual implants do not favor the transmission of this type of signal, reason for which they are more vulnerable in front of the mentioned degenerating agents.

It is well known the fact that the crystals resonating action increases between some limits also because of the increase of their temperature. The frequency normally generated whenever a subject drinks warmer liquids is consumed, a part for regenerating the parodontal tissue and another part for forming the tophus. Hence, the importance of periodically removing the formed dental tophus.

It is scientifically demonstrated by the researchers from the University of Gratz that salt crystals have a therapeutic action due to the vibrations generated. It is known that a positive effect is achieved by the vibrations present at the moment when a subject takes a bath in a tub wherein there is much natural salt at the constant temperature of 37°C. It is estimated that the water temperature shall be constant in order that the vibrations should have a constant frequency. This scientifically demonstrated thing is also applied in case of the buccal cavity where, salt crystals will favour these frequencies normalising the cell equilibrium affected due to the ion changes produced by the microbes that are electrically charged.

There are studies that show that the calcium ion resonate to a 33.3 Hz frequency. This ion is present in any dental and peridental tissues. These oscillations of the mineral particles can exist by the action thereon of some electromagnetic waves frequently issued by various artificial sources, said waves putting them in a synchronous oscillation condition, to specific beneficial biocompatible frequencies.

The experimental results to this effect were also obtained by K.Imamura in 1991 who, by placing plants into simple tap water and into tap water in which some minerals were put, has ascertained that after three weeks, the plant in the non-treated water dried, while the plant in the water with minerals was still green. It was concluded that the emission in infrared, within the wave length spectrum: 2,000-25,000 nm has beneficial vibratory effects on the ions at the cellular membrane level, balancing the ion exchange between the outside and inside of the cell and making the cellular membrane more permeable to nutritive substances, that lengthens the cell life. This effect is also manifested in animal and human tissue cells, as shown by the researches.

WO 02/102457 A1 describes an apparatus to apply an electromagnetic field to modulate the immune system having an electromagnetic field generating device adapted to produce a constant electromagnetic field of less than 1 mT at a radiation frequency between 7 Hz and 8 Hz.

EP 2 074 958 A2 describes a dental implant and the application of a magnetic field for cell regeneration.

### Summary of the Invention

The aforementioned problems are obviated by a system for increasing organic cell regrowth according to the appended independent claim. Preferred embodiments are illustrated in the dependent claims.

The inventor has concluded that titanium, as metal used for implants, has the advantage of workability and a higher elasticity; the zirconium oxide has the advantage of biocompatibility, of favouring the transmission of the regenerating frequencies, and implicitly of a lower reluctance and as such an implant that is called hybrid, which succeeds to combine these two metals, namely having some items made of titanium and others made of zirconium oxide, will be superior to the one made only from titanium or zirconium. Then, if the zirconium to titanium ratio increases, the biological qualities of the implant, namely the osteointegration, will also increase. By varying the ratio between the mass and shapes of the items made of titanium alloys and the ones made of zirconium oxide, used for carrying out the same implant, the elasticity thereof is also varied. As such, there can be carried out an implant adapted to the jaw or mandible portion wherein it is to be integrated.

The tissues regenerating capacity in the affected region depends on the subject's age, his general health condition, health condition of tissues in the implant region, on the capacity of said region to emit regenerating frequencies, implicitly on the tophus volume, on the adjacent teeth condition, on the number and quality of the previous implants, on the osteointegration properties of the new implant. For this reason, in order to accelerate the osteointegration there shall be taken additional steps to allow the increase the regenerating frequencies amplitude. Even the implants that can be defined at a given moment as being successful, in time suffer from resorption problems that are to be slowed down.

In the course of researching optimal regenerating frequencies amplitudes, the inventor has established a surprising effect achieved by an electromagnetic (EM) field generating device, which subjects tissue at the cellular level to a constant EM field in the frequency range of 7 Hz to 8 Hz. Corresponding tests have been conducted independently and are presented hereafter.

The inventor has moreover established a direct relationship between the EM field and the molecular structure of water within the region subjected to the EM field, wherein water maitained in regulated form, that is according to a specific three - dimensional arrangement which is maintained by incorporating the water in some regulate minerals such as sillicates or crystals, further increases the cellular regeneration. This is in opposition to the normal, irregular spatial arrangement of water which acts as a degenerative agent when contained in non - regulate minerals such as dental plaque, dust on the face or the like, and which is inhibiting the regenerative process.

A method of increasing organic cell regeneration in a region of a subject, not according to the present invention, comprises the steps of subjecting cellular tissue in the region to a constant electromagnetic (EM) field of less than 1 mT and having a radiation frequency between 7Hz and 8Hz with an EM field generating device ; and if the subject region is dehydrated or includes unregulated minerals apt to disturb the resonance of water subjected to the EM field, locating a resonance medium substantially at or adjacent the region, wherein the resonance medium comprises a composition which is adapted to resonate at the frequency of an electromagnetic field.

The composition is preferably a biologically active composition containing at least one hydrated mineral. The mineral may be hydrated by regulated water maintained in a constant spatial structure, or by regulated water incorporated in a non - destructible matrix.

The at least one hydrated mineral preferably has a particulate dimension in the range of 20 to 150 µm, to facilitate its integration in the resonance medium.

The mineral is preferably one or a combination selected from the group comprising silicates, crystals, quartz, opal, orthorhombic calcium carbonate, octacalcium phosphate, natural salt crystals, volcanic rock, ground animal horns. The resonance medium is preferably selected from the group comprising a surgical and/or prosthetic implant, a tooth and/or gum guard, a tooth bar, a jaw or head covering, a full or partial face or head mask, a blanket, a pillow.

In an embodiment, the resonance medium may be a container made of a polymer material having a polarization higher than, or at least equal to, that of polyethylene, the composition being comprised therein so that the at least one mineral does not come into contact with tissues adjacent the resonance medium.

In an embodiment, the resonance medium is preferably a layered structure, having the at least one hydrated mineral sandwiched as a layer between polyethylene layers.

Preferably, the EM field is generated as a pulsed signal having a substantially rectangular waveform. The EM field preferably has a radiation frequency of substantially 7.69 Hz. The EM field is substantially 0.75 mT.

In an embodiment the step of subjecting the region to a constant EM field further comprises connecting the adjusted EM field generating device to the resonance medium.

According to the present invention, there is provided a system for increasing organic cell regeneration in a region of a subject, comprising an electromagnetic (EM) field generating device adapted to produce a constant EM field of less than 1 mT at a user - adjustable radiation frequency between 7Hz and 8Hz; and optionally, if the subject region is dehydrated or includes unregulated minerals apt to disturb the resonance of water subjected to the EM field, a resonance medium for locating substantially at or adjacent the region, wherein the resonance medium comprises a composition which is adapted to resonate at the frequency of the EM field generated by the device.

Preferred embodiments and characteristics of integers of the system are substantially as described hereinbefore for the previous exemplary method.

A method of increasing organic cell regeneration in a region of a subject, not according to the present invention, comprises the steps of sampling the region from the subject and obtaining a primary cellular line therefrom, subjecting primary cells of the cellular line to a first constant electromagnetic (EM) field of less than 1 mT and having a radiation frequency between 7Hz and 8Hz for a first period of time, subjecting primary cells of the cellular line to at least a second constant EM field of less than 1 mT and having a radiation frequency between 7Hz and 8Hz for a second period of time substantially equal to the first period of time, wherein the frequency of the second EM field is incremented or decremented relative to the first EM field, for each period of time, determining the rate of cellular regeneration in the sample over the period, and adjusting an EM field generating device to emit an EM field at the first or the at least second frequency providing the highest rate of cellular regeneration.

An embodiment of this method may comprise the further step of locating a resonance medium substantially adjacent the region, wherein the resonance medium houses a composition which is adapted to resonate at the frequency of the EM field, when the subject region is dehydrated or includes unregulated minerals apt to disturb the resonance of water subjected to the EM field.

Preferred embodiments and characteristics of integers of this method are substantially as hereinbefore described in connection with the first method illustrated herein.

According to another aspect, there is provided a resonance medium in the form of a dental implant made as a cylindrical or slightly frustoconical bolt provided with an upper portion serving both for insertion into the jaw bone and to secure the prosthetic blunt into it, a median portion provided at its outer side with a relief drawing (a₃), for example a thread, for ensuring primary retention and possibly, with a leading head at its lower side, characterized in that within some first embodiments it consists of a central hollow rotation outer body (7, 19) which axially includes in a first embodiment a central rod (1) provided with an upper head (b₁) or, in another embodiment, a central nut (7, 19), the rod (1) being provided at the upper head (b₁), respectively the central nut (7, 19) being provided with a profiled cavity (b₃, c₄) for entraining the implant in a rotary motion with a view to inserting it and, axially, with a threaded cavity (b₄, C₆) for securing the prosthetic blunt therein, said rod or said central nut being secured inside the central body (7, 19) by screwing, adhering, welding, anchoring pin, Morse cone or any other means allowing a solid anchoring thereof to the revolution axis of the central body, the central body (7, 19) being also provided with an axial central space (a₄, c₁) wherein there will be disposed even from the manufacturing process various elements made of biologically active compositions, within another embodiment, in its lower side, the central body (16) being closed with a wall obtained by mechanical machining or casting, with a plug (17), a self-threading head (14') or with a screw (20) with the role of securing the central nut (19), also at its lower end, under the central body, the rod (1) being further provided with a self-threading leading head (2).

In an embodiment of the dental implant, for the purpose of preventing the periimplantar infections, all the connections between the metal constituting the rod and the central body made of zirconium alloy are arranged below its upper third.

In another embodiment of the dental implant, for the purpose of obtaining an elasticity in compliance with the jaw portion to be inserted, the ratio between the mass and number of metal alloy elements and the mass and number of zirconium oxide elements will be varied.

According to a different aspect, there is provided a composition comprising crystalline structure minerals such as opal, capable of lowering the magnetic reluctance of a dental portion and/or of resonating with the electromagnetic frequencies in the buccal cavity, characterised in that for carrying out intra-oral devices, namely healing/cicatrizing blunts, temporary prosthetic blunts, guards, radicular pivots, immobilisation bars and incrustation, crowns and bridges said composition will be used incorporated in polar polymers of a polarity at least equal to the one of polyethylene, preferably an acrylate; the composition may also be used as an ingredient for obturation materials.

In an embodiment of the composition, for inserting into the dental implants or for achieving intra-oral devices, the composition may include human or animal tooth ground as fine as opal and admixed therewith in equal ratios.

According to a further aspect, there is provided a composition comprising minerals having a crystalline structure capable of reducing the magnetic reluctance of a dental portion and/or of resonating with the electromagnetic frequencies in buccal cavity, characterised in that it is obtained of natural salt, volcanic rocks and/or animal horns, alone or in admixture, meant to be used for making pillows or coverings for head and/or jaw.

According to a different aspect, there is provided a process for obtaining devices with the composition as described hereinbefore, such as healing/cicatrising blunts and prosthetic blunts, including guards, characterised in that the active crystals are admixed with granules of high polarity polymers at the melting temperature specific to said polymer, then they are regranulated by breaking the thus obtained block, thereafter, in order to obtain blunts for implant, the granules in molten condition will be pressed in die, while for obtaining guards, the obtained granules are hot pressed between two or more sheets of polar polymer depending on the necessary rigidity, thus obtaining a sandwich-like product of which the final product individualised for each subject is obtained.

According to a further aspect, elements are provided, which are made of the composition as described hereinbefore, characterised in that, in case when arranging inside the teeth or implants, they may be as powder or pressed in the shape of the cavity formed inside the tooth or implants, possibly in admixture with a polar polymer.

According to yet another aspect, devices are provided, characterised in that the ones having intra-oral character, namely the temporary blunts, guards as well as the outer ones, namely coverings for head and jaw will be provided with thermostat-controlled heating systems that may ensure a temperature ranging from 50 to 55°C as well as with coils excited form an electronic device for generating biocompatible frequencies, known per se, said coils being inserted in front of the said prosthetic works.

According to another aspect of the present invention, there is provided a material for radicular or coronary obturation, characterised in that, when obtaining the same there are added 20% by weight of opal powder or human or animal tooth ground up to a particle size from 20 to 150 µm.

The dental implant and set of medical elements and devices disclosed are mainly usable for accelerating the osteointegration thereof, according to the principles of the present invention described hereinbefore. However, the set may also be used for accelerating the osteointegration of another type of implant, but it may also be used by persons that previously have already been inserted another type of dental implant, by the ones suffering from chronical parodontopathy as well as by the ones in which the tissue resorption is due to the ageing effects. Also, the set may be used by the persons not having dentition problems, but who want to maintain a natural healthy dentition. The principle at the basis of the invention is developed in order to be applied in stomatology, but there will result from the decription, the fact that it may be used in other fields of the medical sciences.

In the present case, element means a single product made up of a composition that contributes to accelerating the implant integration, said composition being used as loose powder or powder pressed in different shapes to be used as such during the medical acts. Medical device means a unitary assembly made up of more distinct items that may include the composition. From case to case, the medical device may also be connected with other annexed devices with a view to activating it.

The technical problem solved by the present invention consists firstly in achieving an improved regeneration of tissue at the cellular level, and secondly to develop a resonance medium, particularly an implant, with high biocompatibility and regenerating character which, by its construction allows, during its whole life, the amplification of the regenerating frequencies favourable to the affected tissues. If regenerative signals are however weak, or practically absent, it is necessary to enhance them on a natural and/or artificial way, up to an optimum level which should allow both the acceleration of osteointegration of said implant and also maintaining permanently the health of tissues adjacent to the implant, as far as possible for the whole implant life.

The dental implant shall allow to combine the favourable characteristics of the two metals mentioned above, namely the workability of titanium and its alloys with the biocompatibility of zirconium oxide. In other words, it shall be a hybrid implant, with increased biointegrability to be implanted in two stages and to be carried out with an elasticity adapted to the jaw portion in which it will be inserted. Also, even the implant shall contribute, by its own biophysical properties, to accelerating the restoration of tissues affected upon its insertion and implicitly to the osteointegration thereof. In order to enhance the regenerative signals emitted by the tissue or to create artificial signals sufficiently strong and of the adequate frequency - for the case when the autologous signals emitted by tissues are practically missing - there appears the necessity of creating an adequate set of elements and devices to be employed periodically, by the patient, for long or short periods of time, to accelerate the implant osteointegration and/or to restore the adjacent tissues qualities.

The composition employed within the implant may be made of any of the minerals of substances mentioned previously, that have the quality of reducing the local reluctance and, possibly, of resonator for very low frequencies favourable to tissues adjacent to the implant; more exactly it is about mineral compositions including silicates, such as for example opal or some substances such as orthorhombic calcium carbonate, octacalcium phosphate known and already applied in the world, or that will be developed subsequently. As a particular case, the composition may be achieved by grinding a tooth of said patient, or even from another person, up to the particle size of 20 to 150 µm, the tooth being previously extracted for various reasons.

This type of composition, more difficult to be obtained in a large amount, will be introduced even from the manufacturing into the central cavity of the implant as a loose powder or a powder pressed in the shape of some elements with dimensions conjugated to the void inside the implant. From this there can also be carried out some inner prosthetic works that we called intra-oral medical devices, for example cicatrising/healing blunts, temporary prosthetic blunts, guards, dental pivots, bridges etc., by incorporating it into a polar polymer. At the same time upon making cements and materials used for obturating dental works, there can be added up to 20% of their weight, powder opal or human or animal tooth ground up to a particle size of 20 to 150µm.

Of the compositions having the same qualities, but preferably with powder consisting of ground animal horns, with volcanic rock, or natural salt powder, taken alone or in admixture, there can be achieved different external medical devices, such as pillows or various coverings for head and jaw, made by manual work. From case to case, all these devices - more precisely guards, cicatrizing/healing blunts, temporary prosthetic blunts, covering for head and jaw, may be activated at a higher level by heating by means of some thermostate-regulated systems at a tolerable temperature and/or may be provided with some coils by means of which there is locally applied a regenerator magnetic field resulting from a signal generator.

The medical devices that are the subject-matter of the invention are of several general types:
▪ intra-oral - that are carried in the mouth, disposed in the implant or around the implant, for example, blunts, guards, various dental works, bridges, pivots etc; we could also add here the composition as powder or pressed elements of the powder composition to be introduced into the implant or at the root of an extracted tooth;
▪ extraoral or external - coverings for head and jaw, pillows; their use is based on the fact that the regenerating field effect can be ascertained up to 15 mm away from the regenerating composition;
▪ helping - that may be coupled to intra-oral or external medical devices with a view to strengthening their action by heating and/or by connecting them to a controlled electromagnetic signal generator.

The dental implant and the set of medical devices for accelerating the osteointegration thereof, presents the following main advantages:
▪ it combines the biological qualities of some materials with low workability but high biocompatibility, with those of some materials with high workability and high mechanical strength, each of these materials being positioned in key regions and their combination being secure; for example it becomes possible to combine titanium so called grade 5 titanium, to make the self-threading head, with grade 2 titanium, with porous titanium, with zirconium oxide or roxolid elements at the outer shell level. There also results a good primary stability of the implant due to the self-threading head combined with the biological qualities of the outer shell;
▪ the adopted construction also allows to carry out pre-established elasticity implants by varying the ratio between the titanium elements mass and the zirconium elements mass that constitute the implant; thus, depending on the hardness of the bone portion where the implant is to be inserted, we could decide the type of implant adequate to said portion;
▪ it allows the incorporation or attaching active substances favouring the restoration of affected cells and accelerates the osteointegration;
▪ although it includes ceramic materials in a relatively high ratio, the implants permit however to carry out any prosthetic works, of the type possible only for metal implants, such as the screwed in works or rendering the prosthetic blunts parallel in the laboratory;
▪ it is possible to change only the prosthetic blunt in case of fractures, implanting accidents etc, not being obliged to take out the whole implant; also, if there is an acute episode of periimplantite, the final blunt may be changed with a blunt with incorporated minerals, for example in acrylate or another high polarity polymer, for a more rapid regeneration;
▪ there is not possible the formation of electric cells in case of combining zirconium oxide with titanium; nevertheless, some embodiments of an implant in compliance with the present invention are destined to be used in case of some extremely sensitive persons, since the only titanium element is disposed inside an implant body entirely made of zirconium, and the prosthetic blunt may be carried out only of zirconium oxide; also, if different devices generating electromagnetic waves are used, galvanism cannot occur, as the crown head of the implant is made of zirconium oxide;
▪ possible contact regions between the component elements are located below the median third part of the implant, namely where it is no longer necessary to try to treat a possible periimplantite, but it is already demonstrated that the whole implant has to be replaced;
▪ the use of prosthetic and/or healing blunts of a composition adapted to said case favours the osteointegration of the implant and reduction of the possibilities of post-surgery dehiscence and periimplantite occurrence. It is to be mentioned that said blunts may be used not only for the implant claimed by the invention but to any other types of implants already existing on the market;
▪ while the dental implants are made in plants with homologated working conditions, the dental devices to be applied onto the implant may be made both in plants and in dental technique laboratories, there resulting pivots and incrustations, crowns and bridges, prosthetic and healing blunts made of materials with a high degree of polarity, acceptable physionomy and resistance, etc;
▪ incorporating compositions in cements and materials for obturation ensures, besides the role of resonator for the regenerating frequencies, the improvement of the expansion coefficient, consequently, implicitly, a longer duration of resistance of the prosthetic work.

Other aspects are as set out in the claims herein.

### Brief Description of the Drawings

For a better understanding of the invention and to show how the same may be carried into effect, there will now be described by way of example only, specific embodiments, methods and processes with reference to the accompanying drawings in which:

There are given hereinafter more embodiments in connection with Figures 1 to 58 which represent:
Figure 1 - a general axonometric view of a dental implant, in a first embodiment, having the healing blunt mounted;
Figure 2 - a general axonometric view, with partial axial section through the same implant, but without healing-cicatrising blunt;
Figures 3a, b - a longitudinal section about the plane A-A through the implant- Figure 3a, and a top view - Figure 3b;
Figures 4a, b, c - a longitudinal section about the plane C-C, through the central rod of the implant - Figure 4a, top view - Figure 4b, axonometric view- Figure 4c;
Figure 5 - general axonometric view of the self-threading leading head;
Figure 6 - longitudinal section through the central frustoconically shaped shell, in the externally threaded version;
Figure 7 - longitudinal section through a hollow type active element made of a composition accelerating the osteointegration;
Figure 8 - longitudinal section through a healing blunt;
Figure 9 - general spatial view of a dental implant, in a second embodiment, with bayonete-type assembling of the core to the body;
Figure 10 - general view 10a, and longitudinal axial section , 10b, through the body of implant in Figure 9 and 10a, with the view of the securing channels of the central core;
Figure 11 - general view of the central core of the implant;
Figure 12 - general view of the element for accelerating the osteointegration;
Figure 13 - general axonometric view, with partial section of an implant, in a third embodiment, namely with the prosthetic blunt secured by Morse cone;
Figure 14 - longitudinal axial section, according to plane A-A in Figure 16, of an implant, in the third embodiment;
Figure 15 - longitudinal axial section about the plane B-B of Figure 16, of an implant, in the third embodiment;
Figure 16 - top view of the same implant presented in longitudinal section in Figures 14 and 15;
Figure 17 - general axonometric view of the implant shell/body, in the third embodiment, presented in Figure 13;
Figure 18 - general axonometric view, with partial section of an implant similar to the one in the third embodiment, Figure 13, but provided with prosthetic blunt and secured to the implant by means of a usual screw;
Figure 19 - general axonometric view of the prosthetic blunt, secured by screw;
Figures 20 to 23 - general view with sections of another implant, in a fourth embodiment, namely with the self-threading leading head provided with support rod and assembling the blunt to the implant body by screw;
Figure 24 - version of the implant presented in Figures 20 to 23, wherein the upper head of implant is provided with a bottom wall separating the cavity for screwing in the prosthetic blunt to the cavity of the self-treading head rod;
Figures 25 to 27 - the fifth embodiment of the implant, wherein the upper head is provided with a threaded cavity for securing the prosthetic blunt therein and with rod for securing it in the body by means of a pin;
Figures 28 to 31 - other version of the implant of Figures 25 to 27, wherein the upper head of implant is secured to the shell by adequate adhering or welding;
Figures 32 to 34 - version of implant of Figures 1 to 6, wherein the self-threading leading head securing in position to the rod of implant upper head is made by a pin;
Figures 35 to 37 - version of the implant, wherein the body thereof is closed at its lower side by means of a plug, to facilitate the introduction of the regenerating composition therein, and with inner hexagon for achieving the insertion thereof;
Figures 38 to 41 - version of the implant, wherein its body is closed at the lower side by a plug, in order to facilitate the introduction of the regenerating composition therein, and with outer hexagon for achieving the insertion thereof;
Figures 42 to 47 - version of the implant as claimed by the invention wherein its body made of zirconium is provided to the inner side with a metal bush, for example made of titanium, adhered or welded thereto, for assembling the prosthetic blunt;
Figures 48 to 49 - version of the implant, wherein its body is provided to the inner side with a bush, adhered or welded thereto, for assembling the prosthetic blunt, and at the lower end with a hexagonal machining for assembling therein the self-threading leading head;
Figures 50 to 54 - version of the implant of Figures 48 to 49, wherein the self-threading leading head is screwed in the inner bush, to ensure maintaining said inner bush in position;
Figures 55 to 57 - version of the implant of Figures 50 to 54, wherein the self-threading leading head was replaced by a simple cap screw to ensure the maintaining of the inner bush in position;
Figure 58 - principle diagram of an electromagnetic field generating device, apt to generate frequencies biocompatible with the human body.

### Detailed Description of the Embodiments

There will now be described by way of example a specific mode contemplated by the inventors. In the following description numerous specific details are set forth in order to provide a thorough understanding. It will be apparent however, to one skilled in the art, that the present invention may be practiced without limitation to these specific details. In other instances, well-known methods and structures have not been described in detail so as not to unnecessarily obscure the description.

The beneficial action of relatively warm crystals upon the human body is well known. A first explanation is that various minerals or crystals, such as opal for example, activate electromagnetic fields more or less intense in a frequency range beneficial to the human or animal body. A resonance medium comprising a composition that includes at least one mineral or crystal will favour electromagnetic radiations, in a frequency range favourable to its functioning and restoration of the immediately adjacent tissues. It is clear that such a device will also be beneficial to face skin, by generating an age prevention effect, as well as to sinuses, the eyes and still more. Subjects accustomed to one or more resonance media as mentioned above, realise that by wearing it they feel better in several respects. The simplest way of applying these frequencies in the organism is to ask the subject to wear the medium as long as possible, and mainly during the night, for instance as a resonance medium made of a material resonating with some low biocompatible frequencies.

In other words, it is no longer necessary to use only electronic devices capable to emit electromagnetic frequencies beneficial to the human body, but it is also good to create and use static devices which include minerals capable of lowering the local reluctance to, and to resonate with, the biocompatible frequencies, by taking over from the body energy for their functioning. By way of analogy, the clay treatment consisting in applying clay on the gum of the teeth affected by parodontopathy, though unpleasant under many aspects, gives highly positive results, apparently still incomprehensible. The explanation is that the silicates microparticles in the clay composition, activated by the heat in the mouth, amplify the electromagnetic radiations beneficial to the organism.

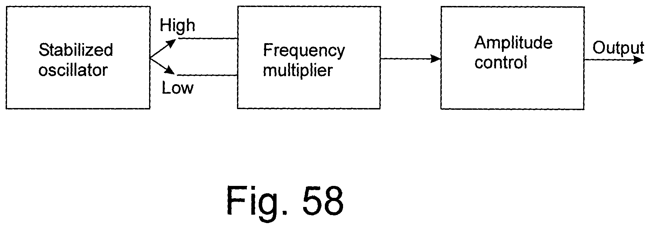

The resonance medium may be connected, for longer or shorter periods of time, as the case may be, by the subject, during pause or rest periods, to electromagnetic (EM) field generating devices for accelerating the integration. The block diagram of such a device is given in Figure 58. A relevant device will be made up of two distinct elements: at least one excitation element, usually a coil, which generates the user - adjustable electro-magnetic field frequency, and the low frequency generator that will have the dimensions of a battery, the subject using the system as an energy healing system. More exactly, the electronic equipment for generating very low frequency magnetic field in the resonance medium, for instance a dental implant or of the tooth region, consists of a microgenerator of functions having an integrated microcircuit at the basis, for example XR2206. The assembly generates a user - adjustable frequency ranging from 0.9 - 75Hz with various signal shapes, preferably a pulse shape having a substantially rectangular waveform. The circuit shall also meet an essential condition with regard to the output signal level. It is known that the healing phenomena have at the basis magnetic inductions of 0.10 to 1 milliTesla (mT), and tests by the applicant have revealed an optimal frequency at substantially 7.69Hz and a magnetic induction level of substantially 0.75 mT.

In certain embodiments, the signal generator will be physically connected to the resonance medium through a very thin coaxial conductor. From the generator, through this conductor, there starts a variable current of the amplitude and frequency decided by sampling the subject's cellular tissue in the region to be treated. Coils will be secured exactly where healing is desired to be accelerated, for example in front of the resonance medium. This device may increase the blood microcirculation both by the electromagnetic oscillation and by thermal way by slightly heating the region to be treated.

It is not however imperative for the device generating the EM field signal to be physically connected to the resonance medium, and alternative embodiments consider electromagnetic field generating devices for use in close proximity to a region of a subject, that are adapted to emit an electromagnetic field onto the region without any physical connection.

Such an EM field generating device according to the present invention was tested with samples of gum cells GI.AC08 cultivated in a medium MCDB153 in a growth medium BC023 over a period of four days.

The device was realised pursuant to the general structure shown in Figure 58 and comprises two copper coils mounted opposite one another, in a configuration referred to as Helmholz coils, so as to create an constant, nearly uniform electromagnetic field of no more than 1mT there-between, driven at set a frequency in the range 7Hz to 8Hz. The device comprises two banks of dipswitches, one for setting the EM field frequency precisely and one for setting the EM field intensity precisely.

The test protocol was as follows:
▪ A tube containing 1.10⁶ cells was defrosted then mixed in 29ml MCDB153.
▪ Petri dishes were then primed with 5ml of the cell suspension, and placed in an incubator at constant 36.5°C, 5% C02.
▪ 5 petri dishes were placed in respective devices 'DM1', 'DM2', 'DM3', 'DM4' and 'DM5' as described above, between the two copper coils, with each device set as a respective frequency within the described range, and all devices set with the same intensity.
▪ 2 petri dishes were placed at least 30cm away from each device, to serve as a control for the testing.

Each device generated a constant electromagnetic field for the entire period of testing, whereby the cells were continually subjected to the electromagnetic field for 4 days. A morphological study of each sample was then performed on the third and fourth days by microscope. From the study, cellular counting was performed in order to determine any cellular proliferation generated by the devices.

Table 1 hereafter shows microscope images corresponding to each sample tested with a respective test device 'DM1', 'DM2', 'DM3', 'DM4', 'DM5' and a control sample, respectively on the third (J3) and fourth (J4) days.

The cellular count on the fourth day of the test was as follows:

| | |
|---|---|
| Control - | 1560000 cells |
| DM 1 - | 1860000 cells |
| DM2- | 2140000 cells |
| DM3- | 2060000 cells |
| DM4- | 2360000 cells |
| DM 5- | 1820000 cells |

The cellular count confirms that the increase in cell number is in the order of approximately 34% for the second 'DM2' and the third 'DM3' devices, however it reaches up to approximately 50% for the fourth 'DM4' device, which was set at 7.69Hz, whereas the first 'DM1' and fifth 'DM5' devices only show an increase of approximately 18%.

**Table 1 - observation of cellular growth on third (J3) and fourth (J4) day**

| | **J3** | **J4** |
|---|---|---|
| **DM1** | 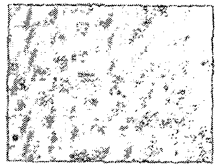 | 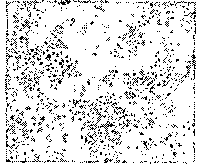 |
| **DM2** | 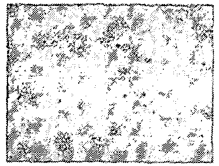 | 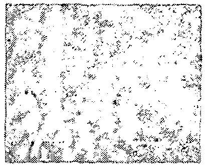 |
| **DM3** | 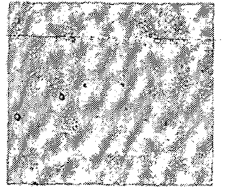 | 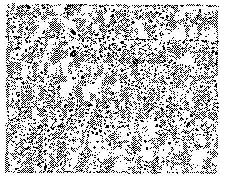 |
| **DM4** | 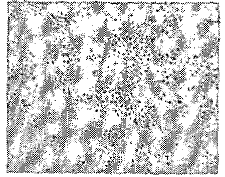 | 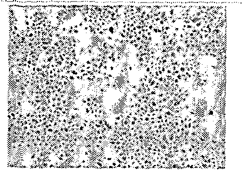 |
| | | |
| **DM5** | 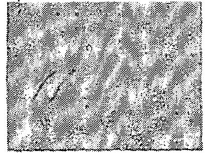 | 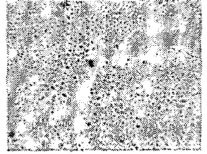 |
| **CTL** | 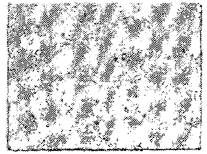 | 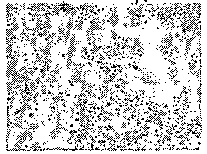 |

After three days of cultivation, with reference to Table 2 hereafter, a zone of confluence was observed in the petri dishes of corresponding to the third 'DM3' and fourth 'DM4' devices, which zones were not present in the other petri dishes. After four days of cultivation, there were still no zone of confluence in the control sample.

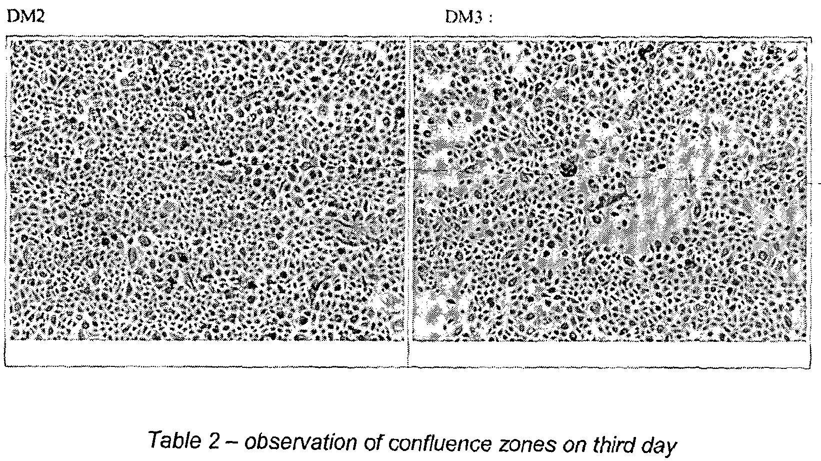

The above tests demonstrate that a device contructed according to the principles and parameters of the present invention demonstrably increase cellular growth, even in the absence of regulated water in the cellular Figure technical solution is presented in many embodiments, mostly in the field of dentistry, but has a same inventive concept equally applicable to very many fields of application far removed from dentistry, encompassing cosmetic treatments, which is the application of an eletromagnetic field onto a mineral, particularly crystalline, composition to favour cellular growth and development.

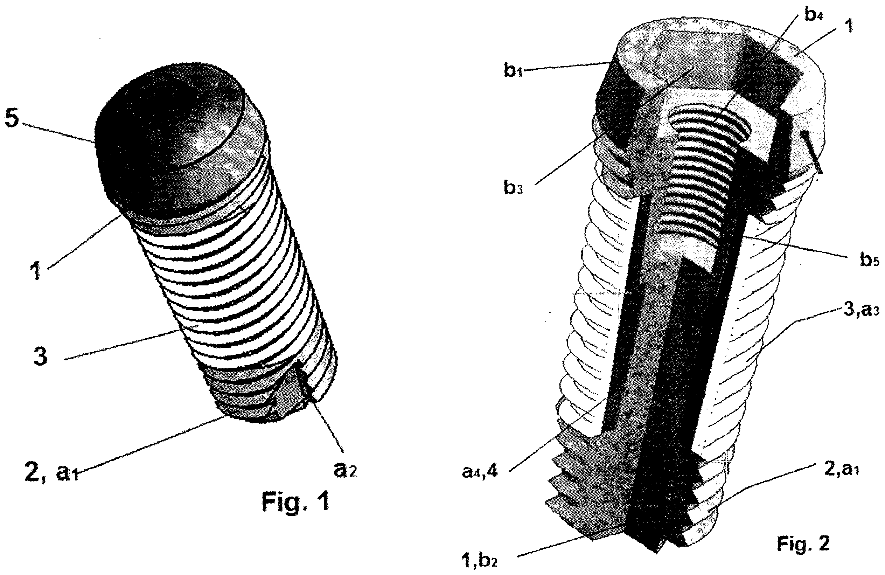

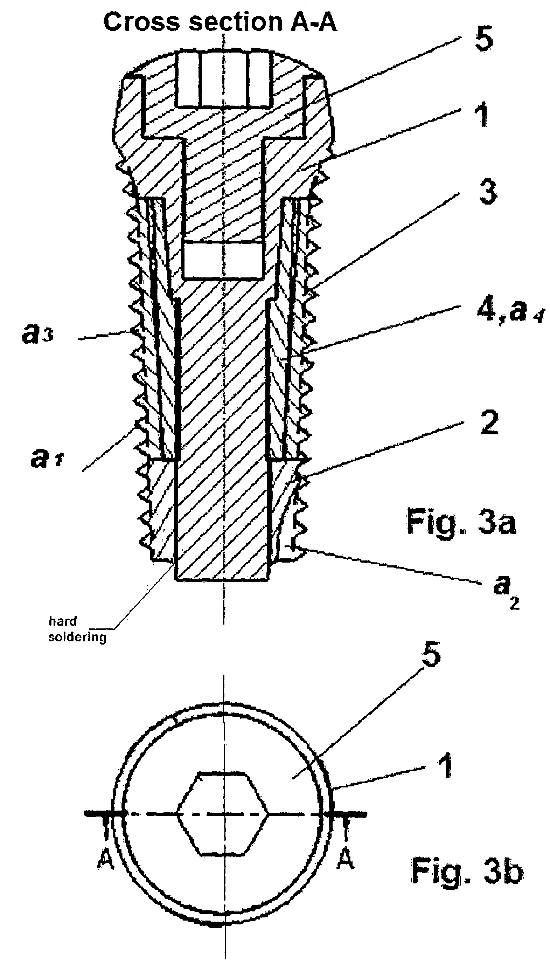

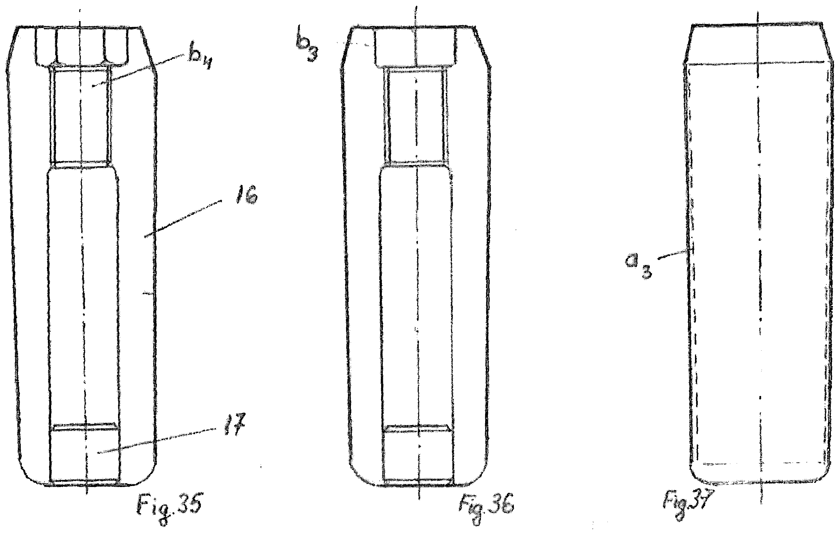

A first conclusion to be drawn is connected to the consistence or hardness of the bone where a certain type of implant is to be inserted. The higher elasticity implants will be used in the rear region where the bone is soft and elastic while in the front region, where the bone is hard, implants of lower elasticity will be chosen. Though maybe more rigid at the beginning, the elasticity of the implant models with rod will vary in time, due to the play occurring between the titanium head and the zirconium oxide body. And this is so because in time, the zirconium oxide will abrade the titanium rod. This adjustment in time of the implant elasticity is in compliance with the modification of the osseous structure. We can say that the implant is adapted to the elasticity modifications of the bone. Due to osteoporosis or even to physiological aging, the bone in time becomes spongious. In time, as the implant elasticity increases, it may adapt to the new situation. As a matter of fact, this advantage - very important - can be found in most implants that are the subject-matter of the invention, except for the ones entirely made of zirconium oxide. The implant entirely made of zirconium oxide is recommended to be used in the front region of the jaws, combining the aesthetic advantage of achieving the cervical region of the implant of zirconium oxide with maintaining a high rigidity, synchronously with the modifications in time of the bones in the front region of the jaws. The implant elasticity may also vary in time depending on the metal the pin is made up of, as well as on the position thereof along the rod, consequently implicitly depending on its length. A pin made of a more elastic metal may allow a higher elasticity of the implant. Then, a general criterion will consist in that, in order to obtain an elasticity in compliance with the jaw portion where the implant is to be inserted, the ratio between the mass and number of elements in the metal alloy and mass and number of elements made of zirconium oxide may vary. In other words, implants that are the object of figures 1, 2, 3, 13, 20, 21 have a higher elasticity, since in the construction thereof more alloyed titanium is incorporated, and the implants that are the object of figures 35, 38, 42 are more rigid, since zirconium oxide predominates here.

Thus, in case of occurrence of periimplantar infection, it was demonstrated that it is possible to sterilize the region affected by infection, provided that in this portion of the implant there does not exist a connection between two components. Also, it was demonstrated that, in case of an implant where the periimplantite decreased below its upper third, the infection cannot be treated and the implant is recommended to be replaced. For this reason, as it can be seen, the implant claimed by the invention, does not have connections in its upper third, fact that confers on it a special mechanical strength.

The special construction of the implant consequently allows the integration therein, even from the factory, of some elements made up of an active composition, but also the temporary addition, for short periods of time, both intra-orally and to the outer side of some dental devices also made of this composition, or of a composition with the same biological characteristics, devices that also have the same role in slowing down the resorption of tissues affected by the implant insertion,

After the implant insertion, the periimplantar healing passes through many stages well-known with regard to chronology and physiology. A stomatologist may reinvigourate this regenerating signals in the period between the implant insertion and the application of the final prosthetic blunt, and by applying some cicatrizing/healing blunts and a temporary prosthetic blunt which should contain the desired substances incorporated in plastics and which, could be connected or not to thermostat-controlled heating devices or to bio**c**ompatible wave generators. After the integration period, the final prosthetic blunt shall be mounted inside the implant. The coexistence of signals permanently generated due to the substances incorporated with signals generated for certain periods of time due to the substances or coils inserted upon a practitionner's decision, will allow an individualized response depending on the subject's reactivity and on the periimplantite occurring hazard.

The composition that is active biologically may be made up of any of the previously quoted minerals or substances which have the quality of lowering the local reluctance, and possibly, of resonator for very low frequencies favorable to tissues adjacent to implant; more exactly, it is about mineral compositions including silicates, such as for example opal, or some substances, such as orthorhombic calcium carbonate, octacalcium phosphate, known and already applied at the moment in the world, or which will be developed subsequently. As a particular case, the composition may also be made by grinding a tooth of said subject, or even from another subject, up to particle dimensions in the range of 20 to 150 µm, said tooth having been previously extracted for various reasons. Since this type of compositions, particularly the ones obtained from a crystal or from a ground human tooth cannot be provided in a large amount, it is recommended to use them intra-orally.

Also as a particular case, the composition may also include ground natural salt crystals, including powder obtained from teeth, horns, human or animal bones, used separately or in admixture. This type of compositions is recommended to be used by external devices.

Also, the compositions meant to be used intra-orally may be employed alone or in admixture with one another and, where appropriate, they may be used as such or embedded in a polar polymer. This polymer is recommended to have a polarization higher than or at least equal to that of polyethylene. In the implant, in order to have a more powerful regenerating effect, the composition is employed, as a rule, simple, as powder, but it may also be employed pressed in the shape of the implant cavity.

The substance to be introduced inside the implant even from the factory and to remain there will be for example opal or calcium carbonate or ground human or animal bone, since it is known that they stimulate the formation of bone. The introduction into some devices of the coils connected to the electronic generator for the purpose of preventing the occurrence of periimplantite is encouraged since a tighter periimplantar ring will be formed that will prevent the occurrence of periimplantite.

Similarly, the intra-oral use composition will also be used as powder if it will be introduced into the space created after the devitalisation of a tooth or at the tooth root.

These compositions may also be employed for preparing materials for radicular or coronary obturations since they simultaneously have three important advantages:
▪ favor the production of the secondary biocompatible radiation;
▪ determine the formation of a new dentine - called secondary dentine;
▪ bring the thermal exansion factor of different materials close to the natural tooth coefficient, thereby avoiding fractures.

In the mixture of materials for obturation, there will be added up to 20% by weight of powder opal or human or animal tooth ground up to a particle size in the range of 20 to 150µm.

The compositions, may also be used to carry out implant elements, for example, having the shape of granules, filaments or tubular or in the shape of a small bolt, depending on the inner implant space. By incorporating into polar polymer such as low or high density polyethylene or in other polymer with a better polymerization degree, the composition may also be used for making implant elements of bigger dimensions, that we called intra-oral dental devices, such as healing and/or cicatrizing blunts or even temporary prosthetic blunts which are to be finally replaced by metal blunts made up of titanium or, preferably, zirconium oxide or roxolid. It is recommended to make these blunts by incorporating minerals into polymers of a polarity higher than polyethylene, and of higher durity, such as, for example acrylate, since the resistance thereof shall be higher, although they are not used for mastication. However, the repeated passing of the alimentary bolus on them causes the destruction thereof.

Rodicular pivots for dental roots, acrylic dental works such as prostheses or fixed works etc may also be made of acrylic material incorporating minerals. There may also be carried out various types of devices used supragingivally, such as for example guards or bars for immobilization of teeth and implants.

Moreover, pillows for sleeping during the night or even various coverings for head and/or jaws may be made of compositions of similar qualities, such as, for example, natural salt, ground animal horns, volcanic rocks etc., for the ones that cannot adapt themselves to wearing a guard. It is to be noticed that the mechanism we described, relating to the treatment with salt is different from the mechanisms used in all other treatments with salt crystals, since the already described treatments are based on the salt contact with the cells - treatments that had an antiseptic effect and the pH adjustment. Our treatment means that the salt, namely other minerals, remains blocked inside the devices and we have no risk of excessive ingeration of salt in case of prolonged treatment compulsory for slowing down tissues resorption.For the inner works, such as guards or dental cements, silicate such as opal may be employed, the amounts of active substance will be less here.

With regard to incorporating the composition in a polymer, the process is the following: the active crystals are mixed with granules of high polarity polymers at a temperature specific to that polymer, then they are regranulated by breaking the thus obtained block. The new granules are processed to the necessary shape by hot or cold die-pressing. For making guards and various prosthetic works said granules are hot pressed between two or more polar polymer sheets, thereby obtaining a sandwich-like product. The advantage of this technique is that the active minerals do not come into contact with the tissues and thus the risks of adverse effects are reduced. It is to be noticed that the temporary character blunts but provided with activating devices and namely with thermostat-controlled heating systems as well as /or coils excited from an electronic device for generating biocompatible frequencies will be connected to these devices for conducting the treatment, and the guards and outer devices such as pillows and head and jaw coverings may be connected to these devices by the subject.

Preferably, all these devices will be carried out with compositions capable of reducing local reluctance and/or of resonating with biocompatible frequencies of 7 Hz if destined to osseous regeneration, 10 Hz if destined for ligament regeneration, 15, 20 and 72 Hz if meant to form new capillaries and 2 Hz, 25 Hz and 50 Hz if meant for nervous regeneration, or 33.3 Hz the calcium resonating frequency if meant to regenerate calcium in tissues.

Though the subject-matter of the present invention is limited only to some problems of the oro-maxilo-facial region, we recommend its adoption by using external devices also specially adapted for solving similar problems in other fields - cosmetics, orthopedic, rheumatology etc. We repeat to this effect the example given at the beginning. Decreasing the blood microcirculation has as an effect the occurrence of wrinkles on the old people face skin. The dead cells are no longer removed and replaced, but they remain in the region causing the occurrence of wrinkles.

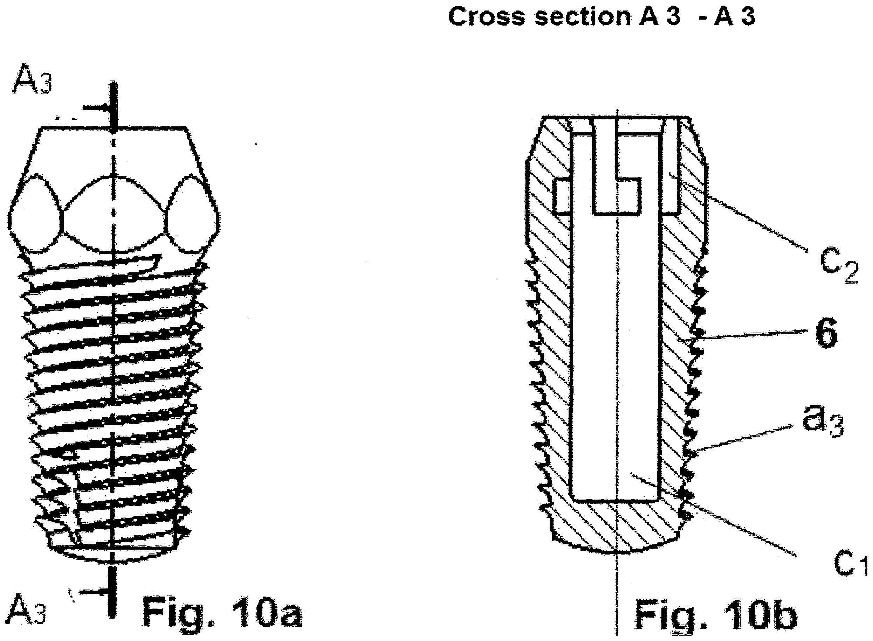

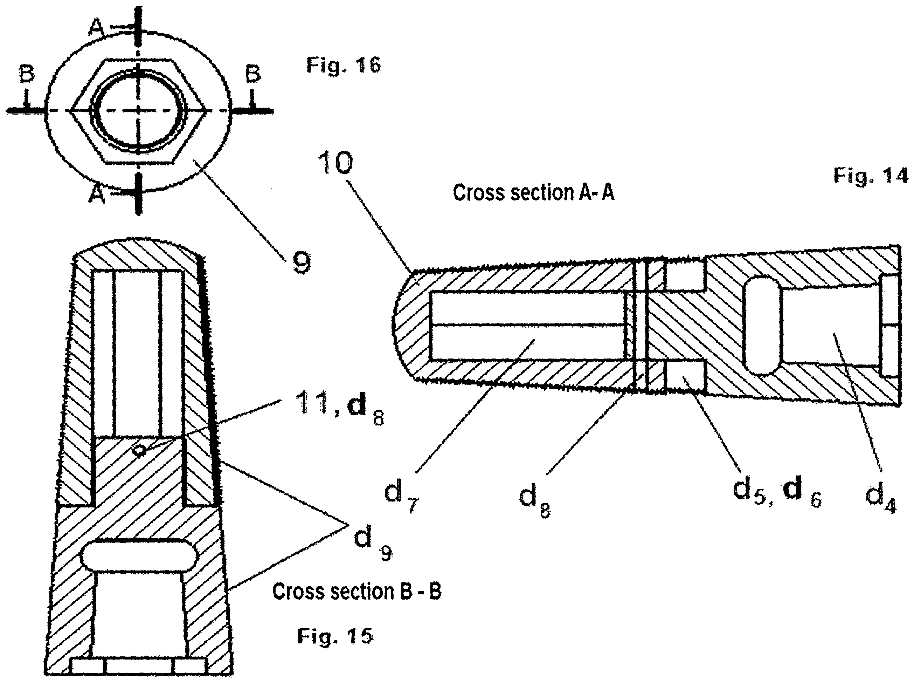

With reference to Figures 1 to 57, there now follows a detailed description of a plurality of embodiments of a resonance medium for use in the specific field of dentistry, however it will be readily apparent to the skilled person, as previously noted, that the single inventive concept dsiclosed herein is capable of application in very many further fields indeed.

An implant shall meet the following technical requirements:
▪ To allow the combination of the titanium and its alloys workability with the biocompatibility of zirconium. This means that the implant elements that will undergo mechanical working - for example, thread of various shapes and dimensions shall be made of titanium and the remainder of the body with role of facilitating the osteointegration will be made of zirconium oxide. Considering things only under this aspect, the zirconium oxide to titanium ratio shall be as high as possible. Furthermore, if a subject proves very sensitive to the titanium presence in the implant, he may have the option of an implant which, though includes titanium in its body, this has to be only at its inner side and cannot come into contact with the surrounding tissues.
▪ To be possible the correlation of implant characteristic of elasticity with the one of the jaw or mandible portion where it is to be inserted. This means that the stomatologist, depending on the concrete case to be solved, shall have the pos suability of choosing a more rigid or more elastic implant, depending on the place this is to be inserted.
▪ In order to accelerate the restoration of tissues affected upon insertion of the implant and implicitly the osteointegration thereof, it shall allow the incorporation therein of one or more implant elements, and/or attaching one/some intra-oral devices made up of a composition known and accepted by the practitioners for the fact that it has a stimulative action on the progenitor cells and produces the restoration of all affected tissues, since it contributes to lowering the magnetic reluctance and/or has the role of resonator for electromagnetic waves existing in the buccal cavity.

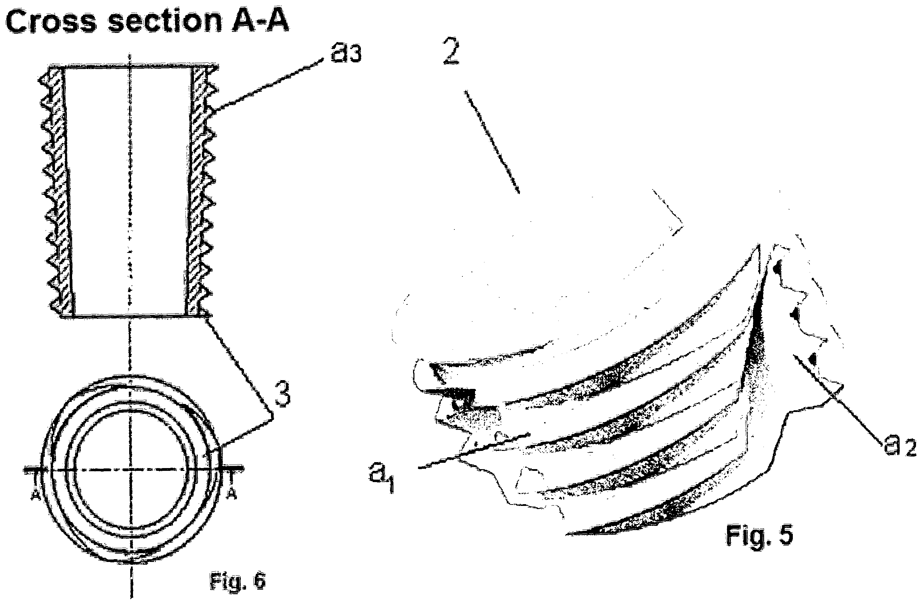

The dental implant with increased biocompatibility is made, in a first embodiment, presented in figures 1 to 8, as an assembly consisting of several items, each having a well established role in implantation itself, in increasing biocompatibility and accelerating osteointegration. This first embodiment includes a central rod with upper head ***1***, hereinafter called briefly only the central rod **1**. It may be made of usual titanium alloys - alloys that are known per se and used at the moment, for example grade 5 titanium, being the most used. The implant may be made of the new type of titanium-zirconium alloy called roxolid. The central rod **1** has a multiple role. First, it has the role of taking over both the torsion stresses occurring during the implanting proper and the compression and bending stresses occurring during mastication. Then, at the lower end of the rod there may be arranged, as in the present case, a self-threading leading head **2** (see also figure 5), made of the same material as the rod in order to avoid the formation of an electrical cell, and at the same time for allowing a lasting adhering - by any adequate process - between the two components **1** and **2**. This solution also allows the combination of grade 5 titanium, recommended for making the self-threading head, with grade 2 titanium usable in the outer shell. The head ***2*** is provided for the same purpose with a cutting thread **a₁**, with one or more beginnings and some cutting teeth **a₂**. Moreover, a self-threading head **2** made of titanium, provided with cutting teeth, has the advantage that it contributes to the increase of the primary degree of stability and will allow the insertion of a predominantly ceramic implant also in regions with fragile bone, such as for example, immediately post-extraction. The third item of the assembly is a central body, or, in other words, a central shell **3** (see also figure 6), preferably made up only of zirconium oxide, in order to facilitate the implant integration into the mandible bone or into the jaw. However, it may also be made of porous titanium, grade 2 titanium or titanium-zirconium alloy, namely roxolid, that was already mentioned. To the outer side of the central body **3**, we can add various compositions for increasing the biocompatibility, for example proteins or various minerals, solution known per se. This shell may have a cylindrical or slightly conical shape and preferably, it shall be threaded to the outer side or at least provided with a relief design **a₃** which should allow the anchoring thereof inside the dental bone, or it may be smooth, if made of porous titanium. The central body ***3*** may surround, at a certain distance, the central rod, so that between it and the rod there exists a small empty space ***a₄*** wherein there may be introduced a composition for accelerating the implant biointegration, said composition being as a powder or, where appropriate, the fourth element of the assembly, namely a hollow part ***4*** may be introduced (see figures 2, 3a and 7), said hollow part being made by pressing from said composition. For the role of part ***4*** in accelerating osteointegration we can call it active pastille. It is true that zirconium does not conduct heat, but the rod is made of titanium and titanium has a good thermal conductivity. Consequently, the heat in the mouth is also transmitted by means of the rod to element ***4*** that has the role of resonator for the regenerating frequencies in the subject's mouth, activating it additionally. The transmission of these frequencies through the zirconium shell is very good, this being the major advantage of zirconium oxide. As a matter of fact, this is the basic mechanism for increasing our implant biointegration. From those mentioned above, it has been ascertained that we have an assembly of parts made - in view of reaching the proposed purpose - of different materials, fact that entitles us to call it a hybrid. After mounting them, the obtained assembly is stiffened through a process of adhering or welding the terminal end of the central rod ***1*** to the front part of the leading head ***2***. The joining may be made by a simple pin, not represented here, which diametrically penetrates through both parts. This version will be further presented within this paper. For avoiding the formation of an electrical cell, this pin will also have to be made up of a material identical to the material for parts ***1*** and ***2***. We mention here, if still necessary, that the ensuring of a joining of more parts through a pin is a process very used in techniques and it is practised in the top field of techniques, in building motor vehicles, in aviation, in armament industry etc. Said pin shall form with the parts it penetrates a tightening fit. It is introduced, for example, by pressing or by other process adequate to said case and may only be extracted by the application of a force at least equal to and of reverse sense.

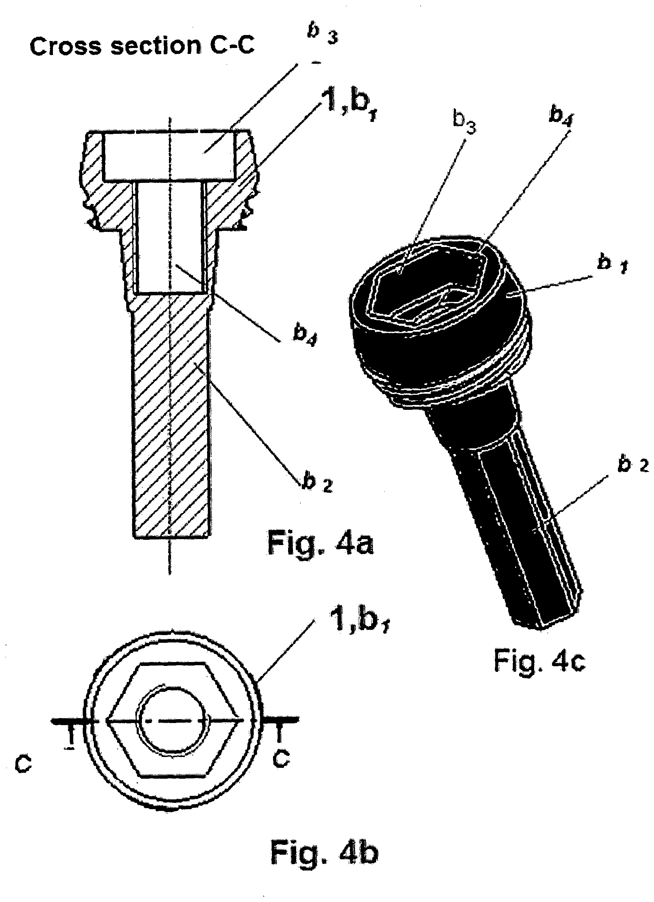

The central rod **1** is provided - see figure 4 - with a polygon-shaped or slightly frustoconical-shaped cylindrical head ***b₁*** lengthened by an polygon-shaped elongate portion ***b₂*** along which all the other items are to be mounted.

The polygon shape of the elongate portion ***b₂*** is first necessary since the self-threading leading head **2** shall be entrained in rotary motion by rotating the rod and second because the leading head **2** and the central shell **3** are threaded externally and the two threads will preferably be arranged one in the extension of the other, which is made easier by rotating them, by 60 degrees about the sides of the elongate polygonal portion ***b₂***. The other end ***b₁*** of the rod ***1*** has, as we have already said, the shape of a slightly frustoconical or even polygonal cylindrical body of a diameter approximately equal to the outside upper diameter of shell ***3***. To its inner side the head ***b₁*** may be provided with an axial hexagonal cavity, ***b₃*** also continuing axially with a relatively short threaded portion ***b₄***, as the prosthetic blunt is to be screwed in therein.

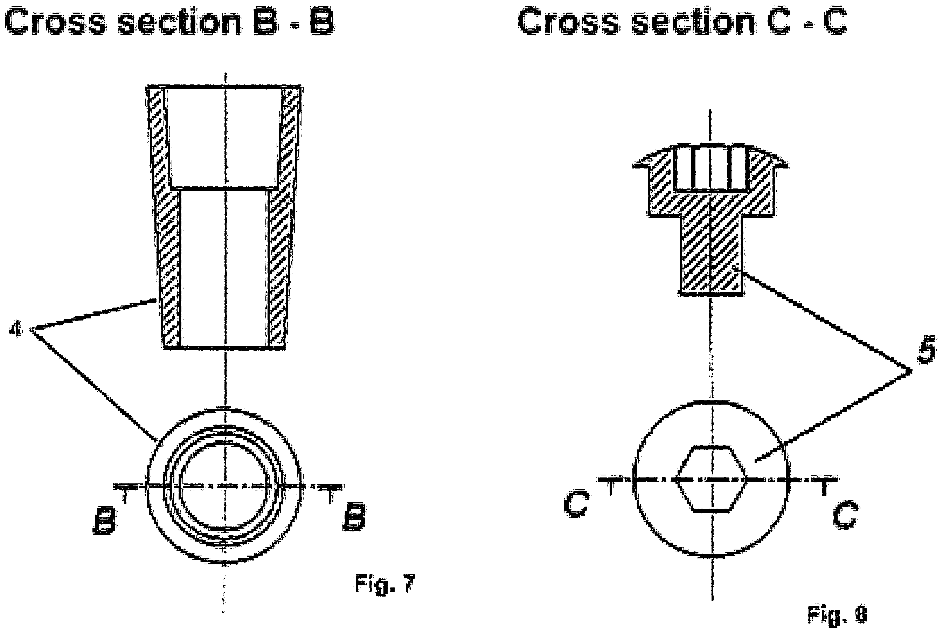

In the hexagonal cavity ***b₃*** of the upper end ***b₁*** of the central rod 1 there may be first arranged a healing/cicatrising blunt **5-** represented in figure 8 - and then, after cicatrising, a prosthetic blunt known per se, whereon the dental crown is to be carried out. They can be tightened on the desired position by means of a screw, solution that is also known. The hexagonal cavity ***b₃*** helps both to insert the implant into the alveolar bone and to mount the prosthetic blunt into the most favourable position with regard to aligning the teeth.

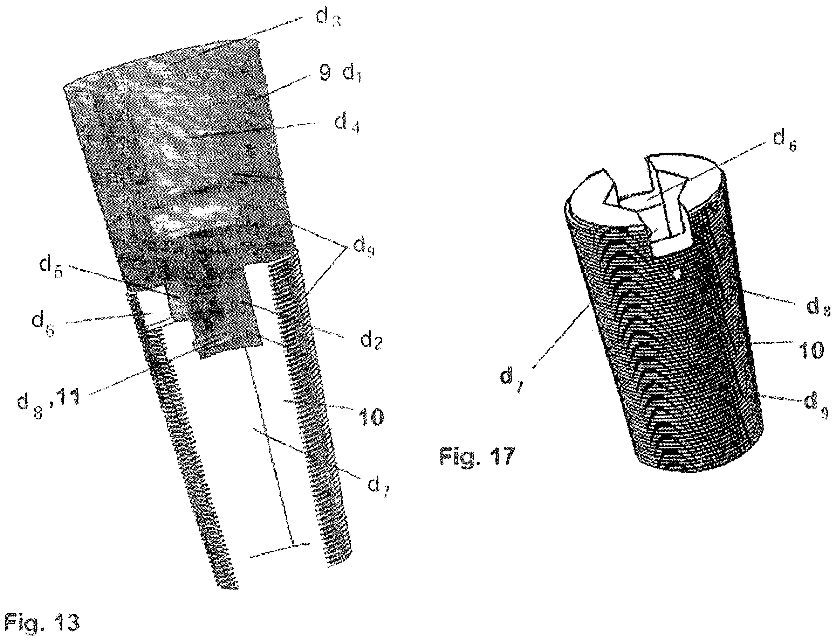

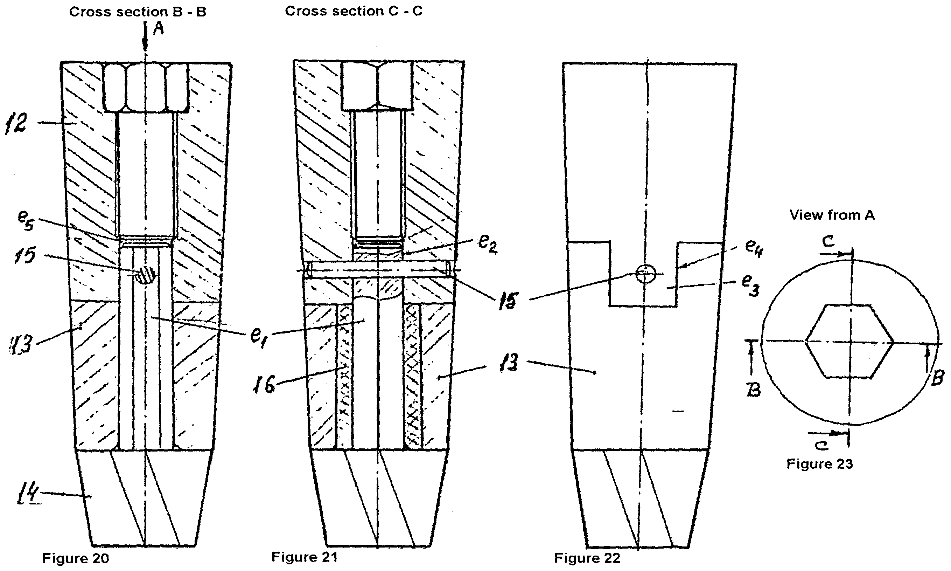

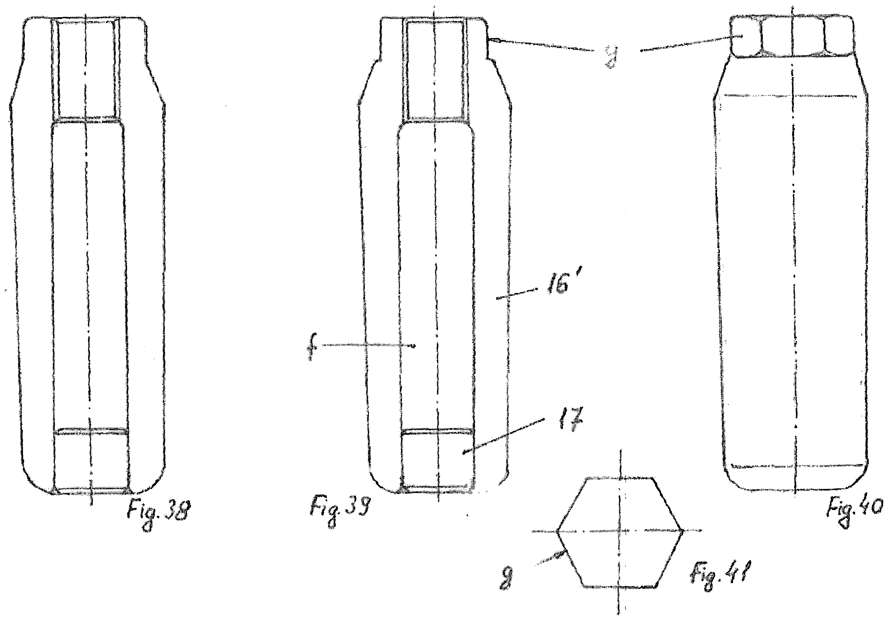

As particular versions of this first embodiment of the implant we can notice that we can also carry out the head ***b₁*** with a hexagonal shape to the outer side which serves both for inserting the implant into the alveolar bone or for mounting a prosthetic blunt provided itself with a corresponding hexagonal cavity - see figures 38 to 41. At the same time, the shell 3 may be anchored to prevent twisting - in case when its inside diameter is larger than the diameter of the circle inscribing the polygon of portion ***b₁***. The thing is carried out very easy by various details of shape such as hexagonal portions or by some fins as the ones represented in figures 13 and 17 and then all the versions presented in figures 20 to 34. This type of implant allows the stomatologist to select, depending on the subject's health condition, age etc an implant with or without the active cartridge ***4***.

This type of implant can also be carried out without the inner space ***a₄*** destined to a composition for accelerating osteointegration, in this case the shell **3** will be made slightly more voluminous. However, we state precisely that all the versions with central rod described here allow to insert therein a composition, only by creating a play between the central rod and the shell made up of zirconium or any other material.

In a similar construction thereof, the self-threading leading head ***2*** may be given up, wherein the outer shell can be closed at the bottom side and the rod ***1*** will also be ensured by a pin- see figure 13 - and will entrain said shell in rotation motion upon implanting, also by means of a polygonal, for example hexagonal cavity.

The second implant embodiment represented in figures from 9 to 12, shows a glass-shaped hollow implant body **6**, namely a body open only at the upper side and closed at the lower side, so that an empty space ***c₁*** is created therein. In its lower side a central core **7** is introduced to be anchored in the implant body by a short simple twisting. For this purpose, the hollow body **6** is provided at the upper side with some short **L**-shaped channels ***c₂***. The central core 7 is provided with some prominences ***c₃*** that may engage in channels ***c₂**.* It can be observed from the drawings that the core **7** is introduced axially into the hollow body **6** and is rotated shortly for anchoring the same in said body. The two components are assembled in an extremely simple way. The composition as powder or as a cylindrical active pastille **8** may be introduced into the created space ***c₁***. The core **7** is made of alloyed titanium or roxolid and is provided at its inner side with an oval or hexagon-shaped machining ***c₄*** made in correspondence with the employed prosthetic blunt. In order to insert the implant into the alveolar bone, the hollow body **6** is provided at its extreme upper side with a hexagon-shaped machining ***c₅*** made on a small portion of its length. However, implanting into the dental bone may be made by using an inner key adequate for the machining ***c₄***. To the outer side, the body **6** is provided with a thread of a known shape ***a₃***. The core **7** is further provided at its lower side with a thread ***c₆***. This helps to secure the prosthetic blunt by means of a screw passing there through. If the prosthetic blunt is also made from zirconium oxide, this type of implant is recommended to persons with completely special sensitivity to metals. The hollow body **6** will be made up of zirconium oxide, roxolid or alloyed titanium. Substances to increase biocompatibility may be added or not to the outer side thereof.

Figures 13 to 17 present the third embodiment of the implant. This implant also includes a central rod with upper head ***9*** to be introduced into the hollow body ***10*** that is closed at the lower side and open at the upper side where the rod is introduced.

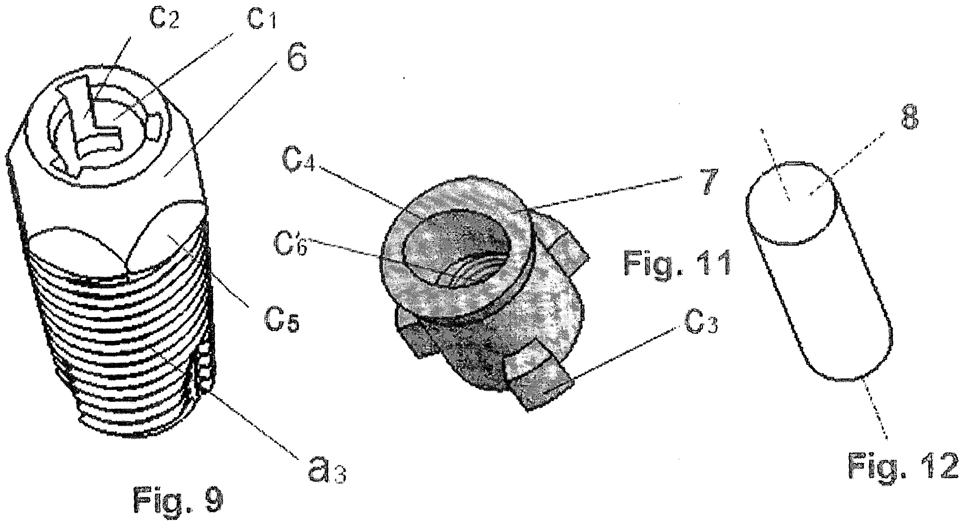

The manner of carrying out allows assembling of the two elements to be made by means of a securing pin ***11***. The rod ***9*** may be made in the manner known from embodiment 1. In other words, it is provided with an upper head ***d₁*** wherein the prosthetic blunt is introduced, and with a polygon-shaped, for example a hexagon-shaped portion ***d₂***, as in the first embodiment. The upper head, for exemplification, is provided with a hexagon-shaped machining ***d₃*** which helps both to insert the implant into the alveolar bone and to secure the prosthetic blunt in the correct position. It further includes a machining ***d₄*** for assembling with a prosthetic blunt by the process with Morse cone. Nothing prevents making a machining for assembling, with the prosthetic blunt, by a thread, exactly as in the first embodiment, as the body ***b*** of the rod in the first embodiment can be processed for assembling with a Morse cone. In the present case, in the rod it is new the fact that at its joining with the hollow body ***10***, the polygon extension ***d*₂** is provided with two diametrically arranged lateral fins ***d*₅**. These fins enter two recesses ***d₆*** made in correspondence, in body ***10*** presented in figure 17. The assembling of rod ***9*** with the hollow body ***10*** will be more steadfast. The body ***10*** is provided, as it could be expected, with a closed axial cavity ***d₇*** in which either a powder composition or an active pastille similar to the one already mentioned in figure 12 will be introduced. We remind that the central rod **9** with the hollow body is assembled by means of a securing pin ***11*.** To this effect, through the two items ***9*** and ***10*** there will be drilled a hole ***d₈*** perpendicular to the longitudinal axis of the implant or laterally, perpendicular to the axis plane, but median to the two fins. It is to be added here the fact that the rod elasticity is depending on the titanium degree of alloying. For example, the grade 5 titanium is more rigid than the grade 2 titanium.

Due to the existence of the two lateral fins ***d₅*** it is no longer necessary for the extension ***d₂*** of the rod to be polygonal. It may also be circular, as the two fins take over the twisting moment and the pin has only the role of positioning, stiffening and securing the assembly. It is the manufacturer's choice to make the rod ***9*** polygonal or not in the section perpendicular to its rotation axis, or round, a version easier to carry out, since the inner cavity ***d₇*** will also be circular. As we have already mentioned, to the outer side the two elements ***9*** and ***1*0** of the implant may be provided with a thread ***d₉***. As construction materials, the rod ***9*** may be carried out of titanium with its alloys, and the hollow body ***10*** may be carried out of zirconium oxide, porous titanium or alloys of titanium with zirconium. Steps may be taken to increase biocompatibility of said hollow body by depositing to the outer side thereof, by the process already known, growth proteins, minerals or other biological stimulants for integration.

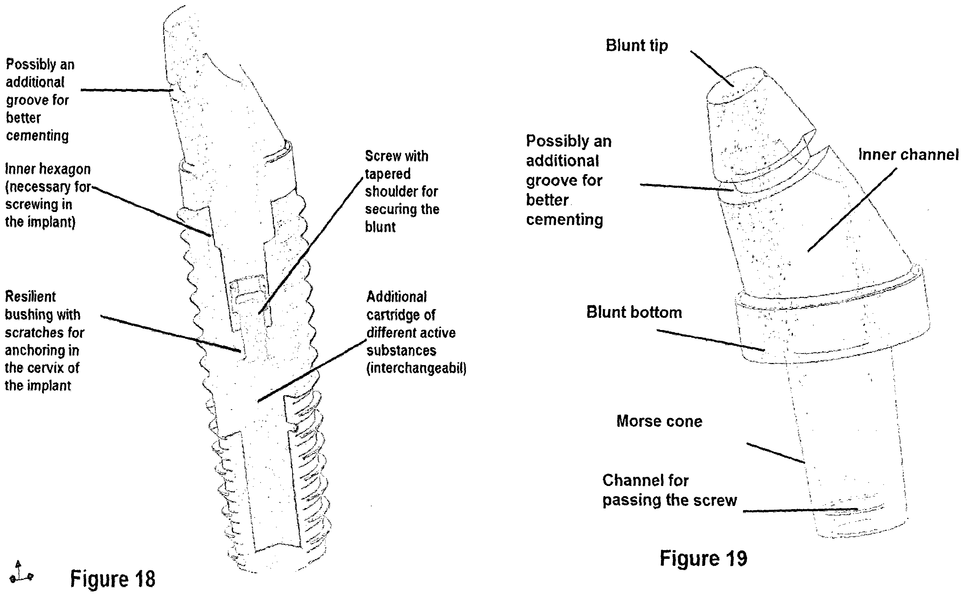

Figures 18 and 19 present a concrete example of assembling a prosthetic blunt with the implant itself. The explanation given on figure 18 are, we hope, sufficiently explanatory. There was chosen the system for securing the prosthetic blunt by means of a resilient bush tightened by means of a screw passing through the inner part of the prosthetic blunt provided with a longitudinal channel. If the inner core/central rod are provided with inner thread (***b₄**, **c₆***) there will also be used a screw and the same shape of blunt.

Of the three given embodiments presented so far, only the first embodiment presents an implant provided with a leading head ***2*.** We also give another embodiment, the forth one of the implant presented in figures 20 to 24. As against the first embodiment, here things are a little reversed. The implant is made of an upper hollow head ***12*** made of alloyed titanium, or roxolid, an intermediary hollow shell ***13*** made up of zirconium or porous titanium and zirconiu with its alloys, a self-threading head ***14*** and an assembling pin ***15*.** To the inner side of the hollow shell ***13*** an active pastille ***16*,** represented only in figure 21 may be introduced. We state precisely, in order to avoid misunderstandings, that in figure 20 the shell ***13*** is secured directly to the rod. This is the situation where the stomatologist considers that for a certain subject it is not necessary to take additional steps for stimulating regeneration. In the new embodiment, the self-threading head ***14*** is provided with a shank ***e₁*** of polygon shaped - for example hexagon shaped - in the cross section. Being provided with a supporting shank the self-threading head ***14*** will be easier to carry out, since there will not be necessary to create auxiliary devices for gripping the same for machining. The upper hollow head ***1*2** with the self-threading head ***1*4** are assembled by means of a securing pin ***1*5.** To this end, the head ***1*2** is provided at its lower side, starting from the end of the inner securing thread of the prosthetic blunt with a polygon shaped hole ***e₂*** made in correspondence with the dimensions of rod ***e₁*** of the self-threading head ***14***.

In figure 22 we give another embodiment of the hollow head **12** and of shell ***1*3.** We have already mentioned this before, when we presented figure 17, but in a reversed version, figure 13, namely when the upper head is provided with a rod. The head ***12*** is made here (figure 22) with two radially arranged lateral extensions ***e₃*** entering two lateral recesses ***e₄*** machined in shell ***13*.** In this case, the twisting of shell ***13*** around the shank ***e₁*** of head ***14*** is prevented when between the shell and the shank ***r*** an active pastille ***16*** is interposed.

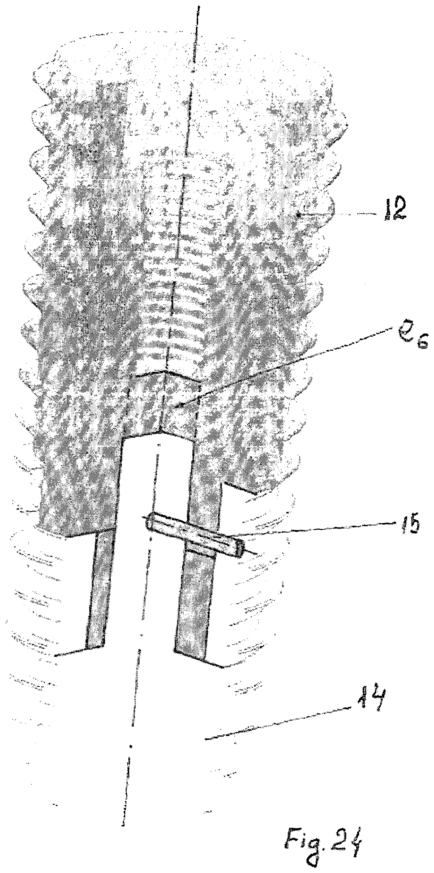

With reference to figures 20 and 21, the distance between the terminal part of the inner thread of the hollow head ***12*** and of the terminal head of rod ***e₁*** of the self-threading head ***14*** may be shorter or longer depending on their length so that a space ***e₅*** may be created there between for introducing any type of active composition therein. This composition may be changed during the healing of the place for insertion of the implant. If it is not desired to create this space, then instead thereof, there may be achieved a separating wall ***e₆*** as presented in figure 24.

We have presented in detail the embodiments so far, fact which will allow us, to present only the main differentiating elements in the following embodiments.

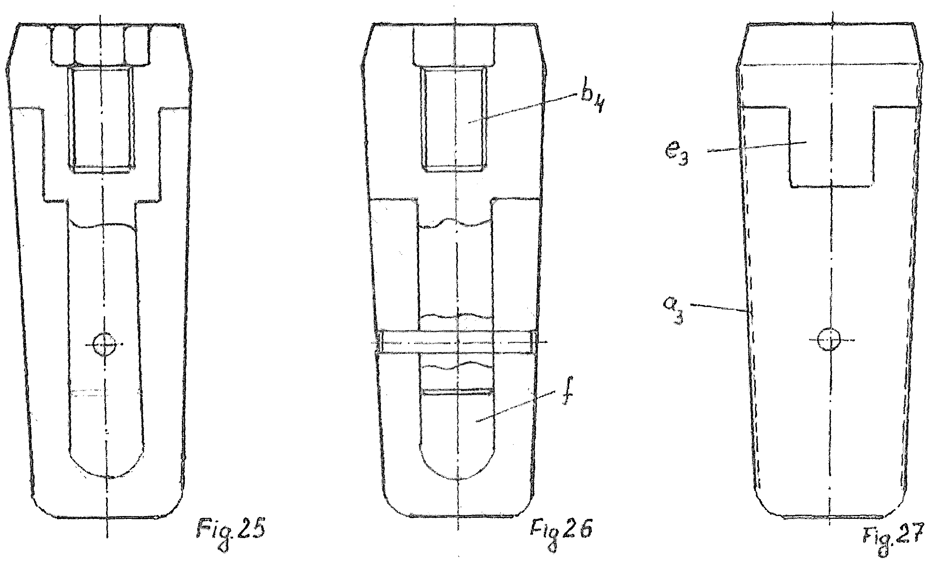

Figures 25 to 27 present the fifth embodiment of the implant according to the invention, where the upper head is provided with threaded cavity for securing the blunt and with rod for securing the same in the shell/body by means of a pin. The rod may also be polygonal, and in this case the fins ***e₃*** may be given up. We recommend however to carry out the two diametrical fins, since this solution may also strengthen the portion where the inner thread ***b₄*** may be carried out. This solution also has as advantages the creation of a longer inner space ***f*** as well as the fact that the whole body of the implant may be of zirconium.

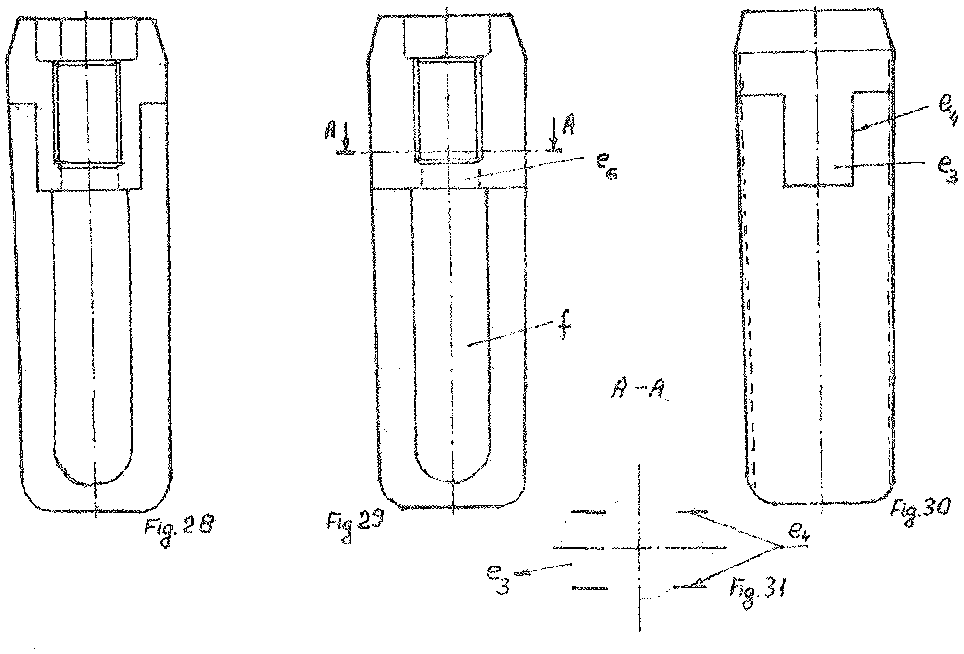

Figure 28 to 31 presents another embodiment of the implant of figure 25 to 27, where the upper head of the implant is joined to the shell by adequate adhering or welding. The implant is secured against twisting only by fins ***e₃*** which enter the cavities ***e₄***. In other words, if there is available an adhesive biocompatible to the human body, the head may be joined with the shell by adhering. Similarly, they may be joined by any welding process which does not cause incompatibilities from a biological viewpoint. In this way, an even longer inner space ***f*** will be created. Where appropriate, the thread base ***e₆*** cover will be given up.

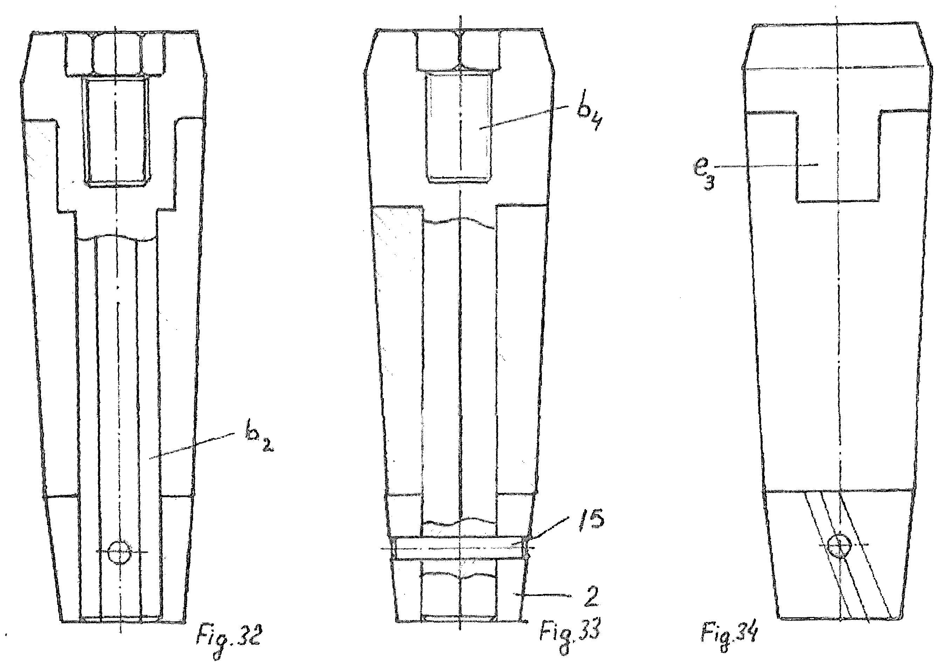

Figures 32 to 34 present another embodiment of the implant of figures 1 to 6, where the self-threading leading head ***2*** securing to rod ***b₂*** of the upper implant head is carried out by the same pin ***15*.** Here there were also maintained the fins ***e₃*** since, as we already mentioned, the threaded hole ***b₄*** will be reinforced.

Figures 35 to 37 present a new embodiment of the implant, namely one where the body ***16*** thereof is carried out entirely of the same material, for example titanium oxide or roxolid. At the same time, this body is closed at its lower side with a plug ***17***, with a view to introducing the regenerating composition easier and it is provided with an inner hexagon ***b₃*** for inserting the same into the dental bone and with thread ***b₄*** for securing the prosthetic blunt. Although we think that it is no longer necessary, we repeat that this body is provided to the outer-side with a relief design ***a₃***. This embodiment has a further advantage, namely the fact that the implant may be carried out easily, both to the inner side and outer side only by mechanical machining on high output automatic machines.

Figures 38 to 41 present a version of the previous implant in figures 35 to 37, according to the invention, where its body ***16***' is closed at its lower side with the same plug ***17*** for introducing the regenerating composition easier, but provided with an outer hexagon ***g*** for carrying out the insertion thereof. In our opinion, the advantage of this version would consist in that it may increase the inner hollow volume ***f***.

At the same time the upper end will have a smaller diameter so as to create easily an adequate prosthetic blunt. It will be sufficient for said prosthetic blunt to cover only one of the outer hexagon sides.

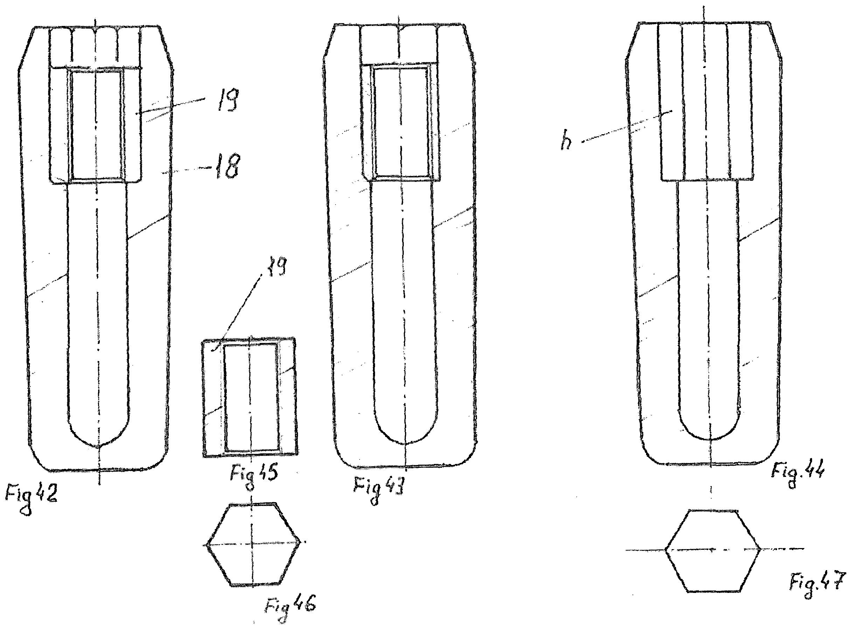

Figures 42 to 47 present a favourite version of the implant where its body ***18*** is provided to the inner part with an inner bush ***19*** threaded to the inner part, but hexagonal to the outer part - in other words, it is identical to a simple hexagonal nut - adhered or welded thereon, for assembling the prosthetic blunt. The bush ***19*** is arranged completely to the inner side of body ***18***, the biocompatibility condition of the adhering solution not being so severe. On the other hand, this implant body may be entirely made of zirconium, since it does not undergo any complex technological machining, for example thread. It is to be noticed that it may also be provided with an inner plug through which the active substance may be filled or, possibly, the welding of the bush to the implant body may be carried out. The hexagonal bush is arranged inside the implant body by providing a hexagonal machining ***h*** at the upper side thereof, said hexagonal machining being carried out in compliance with the hexagonal bush dimensions.

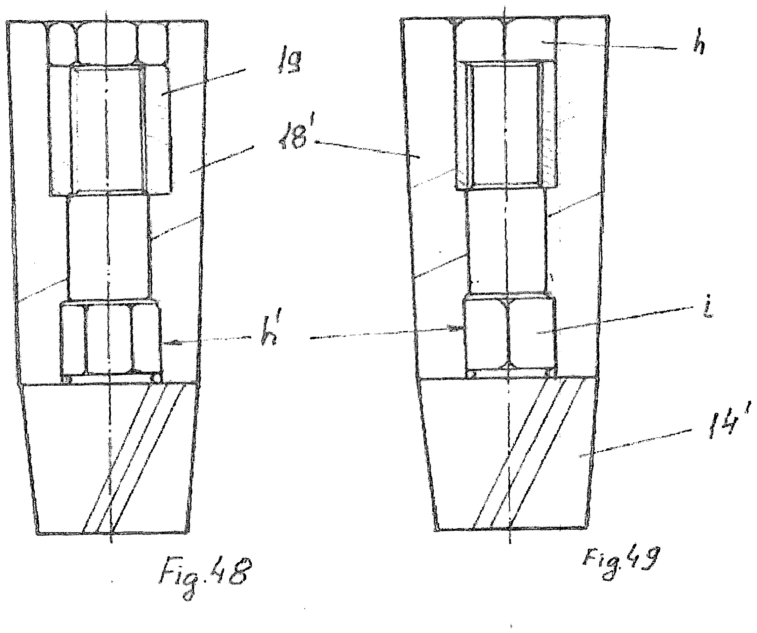

Figures 48 and 49 present a version that is partially similar to the preceding embodiment of the implant, wherein its body ***18'*** is also provided to the inner side with an identical inner bush ***19*** attached to it by adhesive or welded for assembling the prosthetic blunt, but at the lower side it is provided with another hexagonal machining ***h'*** wherein it is also secured by attaching with adhesive or adequate welding, a self-threading leading head ***14'*** similar to the head ***14***, with the only difference that it has a much shorter shank or rod ***i***.

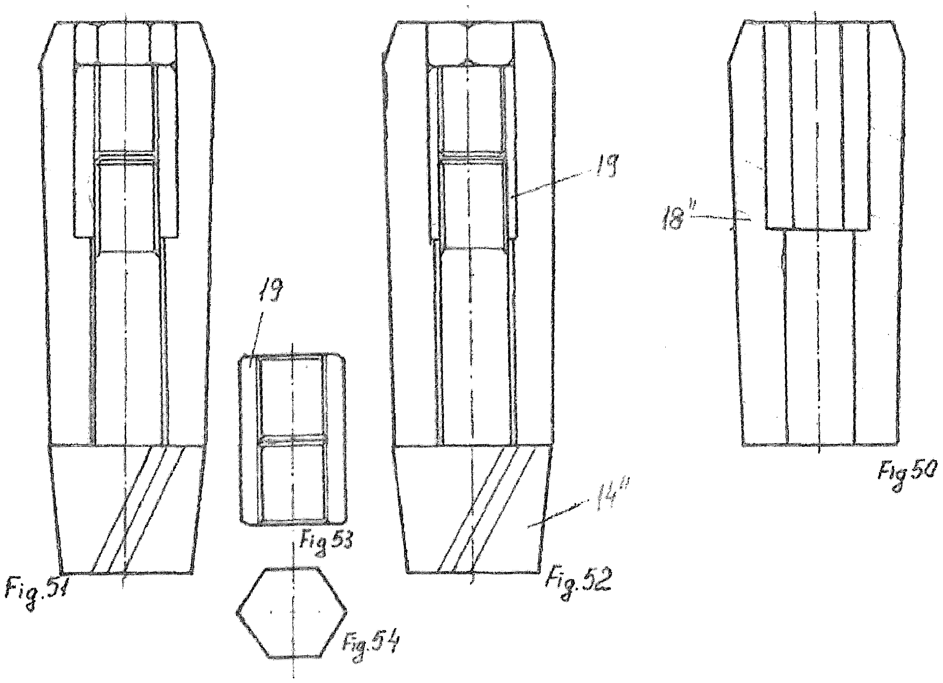

Figures 50 to 54 also present a preferred version of the previous implants in Figures 42 to 49, wherein a body ***18"*** is similar to the two previous ones, but made even simpler, namely without the hexagonal machining ***h'.*** In this case, a self-threading leading head ***14"*** is screwed in the inner bush, in order to ensure the bush being maintained in position. To this end, the leading head is provided with a shank threaded in compliance with the thread of bush ***19.*** The solution is very simple and recommended by us, since we do not have to make any additional works to maintain the inner bush in position. All implants, from 48 to 54 also have the advantage that, by being provided with self-threading head they ensure a very good primary stability, even if they are of ceramics.

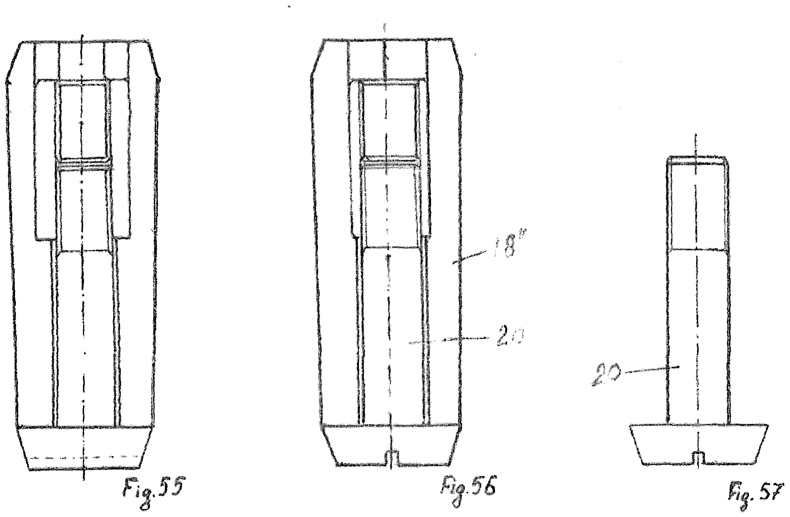

Figures 55 to 57, the version of the implant in figures 50 to 54 wherein the self-threading leading head was substituted by a simple cap screw ***20*** that has only the role to ensure the maintaining of the inner bush ***19*** in position.

In the specification the terms "comprise, comprises, comprised and comprising" or any variation thereof and the terms include, includes, included and including" or any variation thereof are considered to be totally interchangeable and they should all be afforded the widest possible interpretation and vice versa.

The invention is not limited to the embodiments hereinbefore described but may be varied in both construction and detail.

## Claims

1. System for increasing organic cell regeneration in a region of a subject comprising
an electromagnetic (EM) field generating device adapted to produce a constant EM field in said region of a subject of less than 1 mT at a user-adjustable radiation frequency between 7 Hz and 8 Hz, and
a composition to be located substantially at or adjacent said region of a subject, said composition containing at least one mineral, said mineral being hydrated mineral by regulated water maintained in a constant spatial structure or by regulated water incorporated in a non-destructible matrix.

2. System according to claim 1 wherein the at least one hydrated mineral from said composition has a particulate dimension in the range of 20 to 150 µm.

3. System according to claim 1 wherein the mineral from the said composition is one or a combination selected from the group consisting of silicates, crystals, quartz.

4. System according to any of the claims 1 to 3 wherein the EM field generating device comprises at least one coil.

5. System according to any of the claims 1 to 4 wherein the EM field has a radiation frequency of substantially 7.69Hz.

6. System according to any of the claims 1 to 5 wherein the EM field is substantially 0.75 mT.

7. System according to claim 1 to 6 in which said composition comprises crystalline structure minerals capable of lowering the magnetic reluctance of a dental portion and/or resonating with the electromagnetic frequency in the buccal cavity wherein carrying out intra-oral devices, namely healing/cicatrizing blunts, temporary prosthetic blunts, guards, radicular pivots, immobilization bars and incrustation, crowns and bridges said composition is incorporated in polymer materials selected form the group comprising polyethylene, acrylates.

8. System according to claim 7 in which said composition for inserting into dental implants or for achieving intra-oral devices may include human or animal tooth ground and admixed therewith in equal ratios.

9. System according to claim 7 in which said composition is used as an ingredient for obturation materials.

## Patentansprüche

1. System zur Steigerung der organischen Regeneration im Bereich eines Probegegenstandes der
eine Vorrichtung zum Erzeugen eines elektromagnetischen Feldes enthält, die geeignet ist, ein konstantes Magnetfeld in dem jeweiligen Bereich des Probegegenstandes zu erzeugen
von weniger als 1 mT, bei einer vom Benutzer einstellbaren Strahlungsfrequenz zwischen 7 Hz und 8 Hz und
einer Mischung, die sich grundsätzlich am selben Ort oder anliegend an dem betreffenden Bereich befindet,
wobei diese Mischung
mindestens einen Mineralstoff enthält, dieser Mineralstoff regelmäßig mit Wasser in einer konstanten Raumstruktur oder mit Wasser in einer unzerstörbaren Matrix regelmäßig hydratisiert wird.

2. System nach Anspruch 1, wobei mindestens eine Mineralsubstanz der beschriebenen Mischung eine Partikelgröße im Bereich von 20 bis 150 µm aufweist.

3. System nach Anspruch 1, bei dem eine mineralische Substanz der beschriebenen Mischung eine oder eine Kombination aus der Gruppe bestehend aus Silikaten, Kristallen, Quarz ist.

4. System gemäß einer der Anspruch von 1 bis 3, wobei die Vorrichtung zur Erzeugung des Magnetfeldes mindestens eine Spule aufweist.

5. System gemäß einer Anspruch von 1 bis 4, wobei die Vorrichtung zur Erzeugung des Magnetfeldes hauptsächlich eine Strahlungsfrequenz von 7,69 Hz aufweist.

6. System gemäß einer der Anspruch von 1 bis 5, wobei das Magnetfeld hauptsächlich 0,75 mT beträgt.

7. System gemäß einer der Anspruch von 1 bis 6, wobei die beschriebene Mischung kristalline Mineralien enthält, die in der Lage sind, den magnetischen Widerstand eines Zahnabschnitts zu verringern und / oder mit der elektromagnetischen Frequenz in der Mundhöhle, in der die oralen Vorrichtungen arbeiten, in Resonanz zu stehen und nämlich Heilungs- / Heilungsvorrichtungen, temporäre prothetische Abutments, Schutzelemente, Wurzelgelenke, Immobilisierungsstangen und -inlays, Kronen und Brücken, bei denen die beschriebene Mischung das ausgewählte Polymermaterial aus der Gruppe, einschließlich Polyethylen, Acrylate, eingearbeitet ist.

8. System nach Anspruch 7, wobei die Mischung, die zum Einsetzen in Zahnimplantate oder für intraorale Vorrichtungen beschrieben ist, Zahngewebe menschlichen oder tierischen Ursprungs ist oder ein Gemisch davon, in gleichen Anteilen enthalten kann.

9. System nach Anspruch 7, wobei diese Mischung als Bestandteil für Füllungsmaterial verwendet werden.

## Revendications

1. Système de croissance de la régénération organique dans la région d'un sujet contenant
un dispositif de génération de champ électromagnétique adapté pour produire un champ magnétique constant dans cette region
de moins de 1 mT à une fréquence réglable de rayonnement par l'utilisateur entre 7 Hz et 8 Hz et
une composition à être situé essentiellement au même endroit ou près de la région d'un sujet, cette composition contenant au moins une substance minérale, cette substance minérale régulièrement hydratée avec de l'eau préservée dans une structure spatiale constante ou avec de l'eau ordinaire incorporée dans une matrice indestructible.

2. Système selon la revendication 1 dans lequel au moins une substance minérale de la composition décrite a une taille des particules dans la gamme 20 -150 µm.

3. Système selon la revendication 1 où une substance minérale de la composition décrite est une ou une combinaison du groupe composé de silicates, de cristaux, de quartz.

4. Système selon l'une des revendications de 1 à 3, où le dispositif de génération de champ magnétique a au moins une bobine.

5. Système selon l'une des revendications de 1 à 4, où le dispositif de génération de champ magnétique a une fréquence de rayonnement de 7,69 Hz principalement.

6. Système selon l'une des revendications de 1 à 5 dans lequel le champ magnétique est principalement 0,75 mT.

7. Système selon l'une des revendications de 1 à 6 dans lequel la composition décrite comprend des minéraux de structure cristalline, capables de réduire la réluctance magnétique d'une partie dentaire et /ou résonner avec la fréquence électromagnétique dans la cavité buccale dans laquelle il fonctionne les dispositifs intra-oraux, à savoir des dispositifs de guérison/cicatrisation, des prothèses temporaires, des éléments protecteurs, des pivots radiculaires, des barres d'immobilisation et des incrustations, des couronnes et des ponts dans lesquels la composition décrite est incorporée dans le matériel polymère sélectionné du groupe qui comprend le polyéthylène, l'acrylate.

8. Système selon la revendication 7 dans lequel la composition décrite pour l'insertion dans les implants dentaires ou pour les dispositifs intra-oraux peut inclure des tissus dentaires d'origine humaine ou animale et une combinaison de ces deux à parts égales.

9. Système selon la revendication 7 dans lequel ces compositions sont utilisées comme ingrédient pour les matériaux d'obturation.
